# EUROPÄISCHE PATENTSCHRIFT

(11) **EP 3 247 737 B1**
(45) Veröffentlichungstag und Bekanntmachung des Hinweises auf die Patenterteilung: **24.10.2018**
(21) Anmeldenummer: 16701470.3
(22) Anmeldetag: 22.01.2016
(51) Int. Cl.: C08G 65/46, C08G 75/23

(54) **ENTSALZUNG VON POLYARYLETHERN MITTELS SCHMELZEEXTRAKTION**
DESALINATION OF POLYARYL ETHERS BY MEANS OF MELT EXTRACTION
DESSALAGE DE POLYARYLÉTHERS AU MOYEN D'EXTRACTION DE MASSE FONDUE

(30) Priorität: 23.01.2015 EP 15152258
(43) Veröffentlichungstag der Anmeldung: 29.11.2017
(73) Patentinhaber: BASF SE, 67056 Ludwigshafen (DE)
(72) Erfinder: GRAMLICH, Simon, 69493 Hirschberg (DE); STAMMER, Achim, 67251 Freinsheim (DE); ULZHÖFER, Angela, 67065 Ludwigshafen (DE); NIEDERMAIER, Frank, 68526 Ladenburg (DE)
(86) Internationale Anmeldenummer: PCT/EP2016/051374
(87) Internationale Veröffentlichungsnummer: WO 2016/116618

(56) Entgegenhaltungen:
- EP-A2- 0 292 211
- WO-A1-2010/046482
- WO-A1-2014/033321
- CN-A- 102 786 681
- GB-A- 2 376 019

## Beschreibung

Die vorliegende Erfindung betrifft ein Verfahren zur Entsalzung eines salzhaltigen Polymers (sP), das einen Polyarylether und ein Salz (S) enthält, sowie das nach diesem Verfahren erhältliche entsalzte Polymer (eP), das den Polyarylether enthält.

Eine wirtschaftlich besonders bedeutende Gruppe von Polyarylether-Polymeren sind Polyarylethersulfone. Polyarylether-Polymere gehören zur Gruppe der Hochleistungsthermoplasten und zeichnen sich durch hohe Wärmeformbeständigkeit bei guten mechanischen Eigenschaften und einer inhärenten Flammwidrigkeit aus.

Die Herstellung von Polyarylether-Polymeren ist seit langem bekannt. Die Herstellung von Polyarylether-Polymeren erfolgt im Allgemeinen durch Polykondensation entsprechender aromatischer Dihydroxyverbindungen mit aromatischen Dihalogenverbindungen, wobei die Polykondensation in einem aprotisch-polaren Lösungsmittel in Gegenwart von Kaliumcarbonat als Base durchgeführt wird. Die Polyarylether-Polymere fallen im Herstellungsprozess in Form einer Lösung an, die die Polyarylether-Polymere gelöst in dem aprotisch-polaren Lösungsmittel enthält. Das während der Reaktion gebildete Kaliumhalogenid kann mechanisch, beispielsweise durch Zentrifugation oder Filtration, von der Lösung abgetrennt werden, so dass die Lösung und somit auch die nachfolgend isolierten Polyarylether-Polymere nur wenig oder sogar kein Kaliumhalogenid enthalten. Zur anschließenden Isolierung der Polyarylether-Polymere aus dem aprotisch-polaren Lösungsmittel sind im Stand der Technik verschiedene Methoden beschrieben.

Gemäß den in DE 19 57 091 und EP 0 000 361 beschriebenen Verfahren zur Isolierung von Polyarylether-Polymeren, die durch Polykondensation in einem aprotisch-polaren Lösungsmittel hergestellt werden, wird die Lösung, die die Polyarylether-Polymere gelöst in einem aprotisch-polaren Lösungsmittel enthält, in Wasser eingebracht und die Polyarylether-Polymere dadurch gefällt.

Die DE 36 44 464 und die EP 2 305 740 beschreiben ebenfalls Verfahren zur Herstellung von Polyarylether-Polymeren durch Polykondensation in einem aprotisch-polaren Lösungsmittel. Die erhaltene Lösung, die die Polyarylether-Polymere gelöst in dem aprotisch-polaren Lösungsmittel enthält, wird anschließend in ein Fällbad vertropft, das Wasser enthält, und so die Polyarylether-Polymere in Form von Perlen erhalten.

Die EP 0 292 211 beschreibt ein Verfahren zur Herstellung von Arylpolyethern oder Thioethern, wobei ein Bisphenol, ein Bisthiophenol oder ein Hydroxyphenylmercaptan mit einer Dihalobenzoidverbindung in Gegenwart eines basischen Alkalimetallkatalysators in Kontakt gebracht wird, und wobei die Umsetzung in Gegenwart eines Lösungsmittels erfolgt. Die erhaltene Produktlösung wird mit Wasser in Kontakt gebracht und so das Alkalimetallhalid, das sich bei der Reaktion bildet, entfernt.

Die US 4,113, 698 beschreibt ein Verfahren zur Herstellung von Polyetherketonen durch nukleophile Polykondensation von einem Alkalimetall-Bisphenolat mit zumindest einer Dihaloverbindung und/oder einem Alkalimetallhalophenat in einem aromatischen Lösungsmittel. Die erhaltene Reaktionsmischung wird anschließend auskristallisiert oder gefällt und schließlich durch Mahlen auf eine geringe Partikelgröße gebracht und mit Wasser gewaschen.

Die WO 2010/046482 beschreibt ein Verfahren zur Herstellung von Polyetherketonen in Diphenylsulfon unter Erhalt einer Reaktionsmischung, die dann abgekühlt wird, so dass sie fest wird. Die feste Reaktionsmischung wird dann gemahlen und mit Aceton und Wasser extrahiert.

Allen im Stand der Technik beschriebenen Verfahren, bei denen Polyarylether-Polymere durch Polykondensation in einem aprotisch-polaren Lösungsmittel hergestellt werden, ist gemeinsam, dass sie nur einen geringen Gehalt an Kaliumhalogenid aufweisen. Allerdings kann das aprotisch-polare Lösungsmittel nicht vollständig aus den Polyarylether-Polymeren entfernt werden. Diese aprotisch-polaren Lösungsmittel sind folglich auch in Formteilen enthalten, die aus den nach den vorstehend beschriebenen Verfahren erhältlichen Polyarylether-Polymeren hergestellt werden.

Die DE 27 49 645 beschreibt ein Verfahren zur Herstellung von Polyarylethern in einem Schmelzepolymerisationsverfahren durch Polykondensation von mindestens einem Bisphenol mit mindestens einer Dihalogenbenzolverbindung oder von einem Halogenphenol in Gegenwart von wasserfreiem Alkalimetallcarbonat in Abwesenheit von Lösungs- oder Verdünnungsmitteln. Die Reaktion wird in einem Kneter oder in einem Extruder durchgeführt. Die anorganischen Bestandteile, die während der Kondensationsreaktion gebildet werden, beispielsweise Natrium- oder Kaliumchlorid, werden durch Lösen und anschließendes Filtrieren, Sieben oder Extrahieren aus den Polyarylethern entfernt.

Die WO 2014/033321 beschreibt ebenfalls ein Verfahren zur Herstellung von aromatischen Polyarylethern in einem Schmelzepolymerisationsverfahren durch Umsetzung einer Dichlordiphenylsulfonkomponente mit einer Bisphenolkomponente in Gegenwart eines Alkalimetallcarbonats in Abwesenheit von Lösungs- oder Verdünnungsmitteln, wobei die Reaktion in einem Mischkneter durchgeführt wird. Die so erhaltenen Polyarylether-Polymere werden auf eine Teilchengröße von etwa 2 mm vermahlen und zweimal für 3 Stunden bei 80 °C mit Wasser gewaschen, um das als Nebenprodukt gebildete Alkalimetallchlorid zu entfernen. Durch das in der WO 2014/033321 beschriebene Verfahren können jedoch nur 80 % des Akalimetallchlorids aus dem Polyarylether entfernt werden.

Die durch Schmelzepolymerisation hergestellten Polyarylether-Polymere weisen keinen Restgehalt an aprotisch-polarem Lösungsmittel auf.

Die GB 2 376 019 beschreibt ein Verfahren zur Reinigung von Polyketonen. Dabei werden die Polyketone mit Wasser und Extraktionsmittel in Kontakt gebracht. Während des Inkontaktbringens liegt das Polyketon in Form von Pulvern, Pellets oder Granalien vor.

Die CN 102 786 681 beschreibt ein Verfahren zur Reinigung von Polymeren vorzugsweise von Polyetherketonen. Das Polymer wird in fester Form als Pulver, in partikulärer Form oder in kreisförmiger Form eingesetzt. Anschließend wird es mit Wasser als Extraktionsmittel in Kontakt gebracht.

Der vorliegenden Erfindung liegt somit die Aufgabe zugrunde, ein verbessertes Verfahren zur Entsalzung eines salzhaltigen Polymers (sP), das einen Polyarylether und ein Salz (S) enthält, bereitzustellen. Das so hergestellte entsalzte Polymer (eP) soll einen geringen oder keinen Restgehalt an aprotisch-polaren Lösungsmitteln und einen reduzierten Restgehalt an Salz (S) im Vergleich zu den nach den Verfahren des Standes der Technik erhältlichen Polyarylether-Polymeren aufweisen. Das erfindungsgemäße Verfahren und die dadurch erhältlichen entsalzten Polymere (eP) sollen die Nachteile der im Stand der Technik beschriebenen Verfahren und der daraus erhältlichen Polymere nicht oder nur in vermindertem Maße aufweisen. Das erfindungsgemäße Verfahren soll dabei einfach, möglichst fehlerunanfällig und kostengünstig durchführbar sein.

Diese Aufgabe wird erfindungsgemäß gelöst durch ein Verfahren zur Entsalzung eines salzhaltigen Polymers (sP), das einen Polyarylether mit einer Erweichungstemperatur T_{E} und ein Salz (S) enthält, umfassend die Schritte
a) Bereitstellen des salzhaltigen Polymers (sP) bei einer ersten Temperatur T₁, die oberhalb der Erweichungstemperatur T_{E} des Polyarylethers liegt,
b) Inkontaktbringen des in Schritt a) bereitgestellten salzhaltigen Polymers (sP) mit einem Extraktionsmittel (E) unter Erhalt eines entsalzten Polymers (eP), das den Polyarylether enthält, und eines salzhaltigen Extraktionsmittels (sE), das das Extraktionsmittel (E) und das Salz (S) enthält,
wobei der Schritt a) die folgenden Schritte umfasst:
i) Bereitstellen eines ersten salzhaltigen Polymers (s1P), das den Polyarylether und das Salz (S) enthält,
ii) Granulieren des in Schritt i) bereitgestellten ersten salzhaltigen Polymers (s1P) unter Erhalt eines granulierten ersten salzhaltigen Polymers (gs1P),
iii) Inkontaktbringen des in Schritt ii) erhaltenen granulierten ersten salzhaltigen Polymers (gs1P) mit dem Extraktionsmittel (E) unter Erhalt des salzhaltigen Polymers (sP), das den Polyarylether und Reste des Salzes (S) enthält, und eines ersten salzhaltigen Extraktionsmittels (sE1), das das Extraktionsmittel (E) und einen Teil des Salzes (S) enthält,
iv) Erwärmen des in Schritt iii) erhaltenen salzhaltigen Polymers (sP) auf eine erste Temperatur T₁, die oberhalb der Erweichungstemperatur T_{E}, vorzugsweise oberhalb der Glasübergangstemperatur T des Polyarylethers liegt,
und wobei der Schritt b) den folgenden Schritt umfasst:
v) Inkontaktbringen des in Schritt iv) erwärmten salzhaltigen Polymers (sP) mit dem Extraktionsmittel (E) unter Erhalt des entsalzten Polymers (eP), das den Polyarylether enthält, und eines zweiten salzhaltigen Extraktionsmittels (sE2), das das Extraktionsmittel (E) und die Reste des Salzes (S) enthält,
wobei das erste salzhaltige Polymer (s1P) in Schritt i) durch ein Schmelzepolymerisationsverfahren bereitgestellt wird.

Gegenstand der vorliegenden Erfindung ist weiterhin ein Verfahren zur Entsalzung eines salzhaltigen Polymers (sP), das einen Polyarylether mit einer Erweichungstemperatur T_{E} und ein Salz (S) enthält, umfassend die Schritte
a) Bereitstellen des salzhaltigen Polymers (sP) bei einer ersten Temperatur T₁, die oberhalb der Erweichungstemperatur T_{E} des Polyarylethers liegt,
b) Inkontaktbringen des in Schritt a) bereitgestellten salzhaltigen Polymers (sP) mit einem Extraktionsmittel (E) unter Erhalt eines entsalzten Polymers (eP), das den Polyarylether enthält, und eines salzhaltigen Extraktionsmittels (sE), das das Extraktionsmittel (E) und das Salz (S) enthält.
Es wurde überraschend festgestellt, dass mit dem erfindungsgemäßen Verfahren im Vergleich zu den im Stand der Technik beschriebenen Verfahren im gleichen Zeitraum mehr Salz (S) aus dem salzhaltigen Polymer (sP) entfernt werden kann. Das heißt, das Salz (S) kann schneller aus dem salzhaltigen Polymer (sP) entfernt werden.

Überraschenderweise kann mit dem erfindungsgemäßen Verfahren ein Salzgehalt von maximal 150 Gew.-ppm im entsalzten Polymer (eP) erreicht werden. Dadurch wird die Lagerstabilität des entsalzten Polymers (eP) deutlich erhöht gegenüber den Polyarylethern aus dem Stand der Technik, die durch ein Schmelzepolymerisationsverfahren hergestellt werden. Das entsalzte Polymer (eP) besitzt zudem eine gute Schmelzestabilität. Beim Wiederaufschmelzen wird also weder der Polyarylether abgebaut, noch schreitet die Polymerisation des Polyarylethers weiter fort.

Die Polyarylether-Polymere besitzen eine gute Lagerstabilität. Die erhaltenen Polyarylether-Polymere besitzen eine gute Schmelzestabilität. Zudem können die Polyarylether-Polymere häufig als Membranen eingesetzt werden.

Das erfindungsgemäße Verfahren ist außerdem insbesondere geeignet für die Entsalzung von salzhaltigen Polymeren (sP), die durch ein Schmelzepolymerisationsverfahren hergestellt worden sind. Werden in dem erfindungsgemäßen Verfahren durch Schmelzepolymerisationsverfahren hergestellte salzhaltige Polymere (sP) eingesetzt, so weisen die entsalzten Polymere (eP) keinen Restgehalt an Lösungsmittel auf. Somit sind die so erhältlichen entsalzten Polymere (eP) auch für die Herstellung von Formteilen einsetzbar, die für Lebensmittelanwendungen geeignet sind. Die Formteile sind aus toxikologischer Sicht weitestgehend unbedenklich.

Nachfolgend wird das erfindungsgemäße Verfahren näher erläutert.

### Salzhaltiges Polymer (sP)

Erfindungsgemäß enthält das salzhaltige Polymer (sP) einen Polyarylether und ein Salz (S).

Unter "einem Polyarylether" wird erfindungsgemäß genau ein Polyarylether als auch Mischungen aus zwei oder mehreren Polyarylethern verstanden.

Unter "ein Salz (S)" wird erfindungsgemäß genau ein Salz (S) als auch Mischungen aus zwei oder mehreren Salzen (S) verstanden.

In einer Ausführungsform enthält das salzhaltige Polymer (sP) mindestens 50 Gew.-%, besonders bevorzugt mindestens 60 Gew.-%, mehr bevorzugt mindestens 65 Gew.-% und insbesondere bevorzugt mindestens 70 Gew.-% des Polyarylethers, jeweils bezogen auf das Gesamtgewicht des salzhaltigen Polymers (sP).

In einer weiteren Ausführungsform enthält das salzhaltige Polymer (sP) höchstens 99,98 Gew.-%, bevorzugt höchstens 99 Gew.-%, mehr bevorzugt höchstens 90 Gew.-% und insbesondere bevorzugt höchstens 80 Gew.-% des Polyarylethers, jeweils bezogen auf das Gesamtgewicht des salzhaltigen Polymers (sP).

Bevorzugt enthält das salzhaltige Polymer (sP) 50 bis 99,98 Gew.-% des Polyarylethers, besonders bevorzugt 60 bis 99 Gew.-% des Polyarylethers, insbesondere bevorzugt 65 bis 90 Gew.-% des Polyarylethers und am meisten bevorzugt 70 bis 80 Gew.-% des Polyarylethers, jeweils bezogen auf das Gesamtgewicht des salzhaltigen Polymers (sP).

In einer Ausführungsform enthält das salzhaltige Polymer (sP) mindestens 0,02 Gew.-%, bevorzugt mindestens 1 Gew.-%, besonders bevorzugt mindestens 10 Gew.-% und insbesondere bevorzugt mindestens 20 Gew.-% des Salzes (S), jeweils bezogen auf das Gesamtgewicht des salzhaltigen Polymers (sP).

In einer weiteren Ausführungsform enthält das salzhaltige Polymer (sP) höchstens 50 Gew.-%, bevorzugt höchstens 40 Gew.-%, mehr bevorzugt höchstens 35 Gew.-% und insbesondere bevorzugt höchstens 30 Gew.-% des Salzes (S), jeweils bezogen auf das Gesamtgewicht des salzhaltigen Polymers (sP).

Es ist außerdem bevorzugt, dass das salzhaltige Polymer (sP) 0,02 bis 50 Gew.-% des Salzes (S) enthält, besonders bevorzugt 1 bis 40 Gew.-% des Salzes (S), insbesondere bevorzugt 10 bis 35 Gew.-% und am meisten bevorzugt 20 bis 30 Gew.-% des Salzes (S), jeweils bezogen auf das Gesamtgewicht des salzhaltigen Polymers (sP).

Es ist möglich, dass das salzhaltige Polymer (sP) zusätzlich Additive enthält. Geeignete Additive sind dem Fachmann als solche bekannt. Für den Fall, dass das salzhaltige Polymer (sP) zusätzlich Additive enthält, enthält das salzhaltige Polymer (sP) im Allgemeinen 0,01 bis 10 Gew.-% Additive, bevorzugt 0,01 bis 7 Gew.-% Additive und insbesondere bevorzugt 0,01 bis 5 Gew.-% Additive, jeweils bezogen auf das Gesamtgewicht des salzhaltigen Polymers (sP). In einer Ausführungsform enthält das salzhaltige Polymer (sP) keine zusätzlichen Additive.

Darüber hinaus kann das salzhaltige Polymer (sP) eine Carbonatverbindung (C) enthalten. Für die Carbonatverbindung (C) gelten die weiter unten beschriebenen Ausführungen und Bevorzugungen. Für den Fall, dass das salzhaltige Polymer (sP) eine Carbonatverbindung (C) enthält, enthält das salzhaltige Polymer (sP) im Bereich von 0,01 bis 20 Gew.-%, bevorzugt im Bereich von 0,01 bis 5 Gew.-% und insbesondere bevorzugt im Bereich von 0,01 bis 2 Gew.-% der Carbonatverbindung (C), bezogen auf das Gesamtgewicht des salzhaltigen Polymers (sP). Die Carbonatverbindung (C) ist von dem Salz (S) verschieden. In einer Ausführungsform enthält das salzhaltige Polymer (sP) keine Carbonatverbindungen (C).

"Eine Carbonatverbindung (C)" bedeutet im Rahmen der vorliegenden Erfindung sowohl genau eine Carbonatverbindung (C) als auch eine Mischung aus zwei oder mehreren Carbonatverbindungen (C).

In einer weiteren Ausführungsform enthält das salzhaltige Polymer (sP) 50 bis 99,98 Gew.-% des Polyarylethers und 0,02 bis 50 Gew.-% des Salzes (S), bevorzugt 60 bis 99 Gew.-% des Polyarylethers und 1 bis 40 Gew.-% des Salzes (S), insbesondere bevorzugt 65 bis 90 Gew.-% des Polyarylethers und 10 bis 35 Gew.-% des Salzes (S) und am meisten bevorzugt 70 bis 80 Gew.-% des Polyarylethers und 20 bis 30 Gew.-% des Salzes (S), jeweils bezogen auf das Gesamtgewicht des salzhaltigen Polymers (sP). Im Allgemeinen ergeben die Summen der Gewichtsprozente des Polyarylethers, des Salzes (S) und gegebenenfalls der zusätzlichen Additive sowie der Carbonatverbindung (C) 100 %.

Die Viskositätszahlen des salzhaltigen Polymers (sP) liegen im Allgemeinen im Bereich von 30 bis 120 ml/g, bevorzugt von 35 bis 110 ml/g und insbesondere bevorzugt von 40 bis 100 ml/g, bestimmt durch Ubbelehde-Viskositätszahlmessung einer 0,01 g/ml Lösung des salzhaltigen Polymers (sP) in einer 1 : 1 Phenol/1,2-Dichloridbenzol-Mischung nach DIN 51562.

In einer weiteren, bevorzugten Ausführungsform der vorliegenden Erfindung liegen die Viskositätszahlen des salzhaltigen Polymers (sP) im Bereich von 15 bis 900 ml/g, bevorzugt von 22,5 bis 75 ml/g und insbesondere bevorzugt von 26,25 bis 71,25 ml/g, bestimmt durch Ubbelehde-Viskositätszahlmessung einer 0,01 g/ml Lösung des salzhaltigen Polymers (sP) in einer 1 : 1 Phenol/1,2-Dichloridbenzol-Mischung nach DIN 51562.

Im Allgemeinen enthält das Salz (S) ein Kation und ein Halogenid, bevorzugt ein Kation und ein Chlorid. Ein Halogenid wird auch als "Halogenidanion" bezeichnet. Ein Chlorid wird auch als "Chloridanion" bezeichnet.

Unter "ein Kation" werden erfindungsgemäß genau ein Kation als auch Mischungen aus zwei oder mehreren Kationen verstanden.

Unter "ein Halogenid" werden erfindungsgemäß genau ein Halogenid als auch Mischungen aus zwei oder mehreren Halogeniden verstanden.

Die Gew.-% des Salzes (S) in dem salzhaltigen Polymer (sP) können daher über die Messung der Gew.-% des Halogenids, bevorzugt des Chlorids in dem salzhaltigen Polymer (sP) bestimmt werden. Unter den Gew.-% des Halogenids sind die Gew.-% des anionischen Halogens zu verstehen, also die Gew.-% des freien Halogenids und nicht die Gew.-% des polymergebundenen Halogens. Entsprechendes gilt für die Gew.-% des Chlorids. Diese beziehen sich auf die Gew.-% des ionischen Chlors und somit auf die Gew.-% des freien Chlorids und nicht auf die Gew.-% des polymergebundenen Chlors.

Zur Bestimmung der Gew.-% an Halogenid, bevorzugt an Chlorid in dem salzhaltigen Polymer (sP), werden 700 mg des salzhaltigen Polymers (sP) in N-Methylpyrrolidon (NMP) gelöst und die erhaltene Lösung mit einer Essigsäure-/Aceton-Mischung (Verhältnis von Essigsäure zu Aceton: 1 : 1) verdünnt. Die so erhaltene Lösung wird mit Schwefelsäure oder Salpetersäure angesäuert und anschließend mit einer 0,0002 mol/l Silbernitratlösung potentiometrisch titriert, wobei Methylorange als Indikator eingesetzt wird. Als Elektrode dient eine Ag-Titrode der Firma Metrohm.

Über die Gew.-% an Halogenid können anschließend die Gew.-% des ebenfalls im Salz (S) enthaltenen Kations im salzhaltigen Polymer (sP) errechnet werden. Methoden hierzu sind dem Fachmann bekannt. Aus der Summe der Gew.-% des Halogenids sowie der Gew.-% des Kations im salzhaltigen Polymer ergeben sich dann die Gew.-% des Salzes (S) im salzhaltigen Polymer (sP).

Die Gew.-% an Salz (S) in dem nachfolgend beschriebenen vorentsalzten Polymer (veP) und dem entsalzten Polymer (eP) werden erfindungsgemäß in gleicher Weise bestimmt.

Polyarylether sind dem Fachmann als Polymerklasse bekannt. Für den Einsatz im erfindungsgemäßen Verfahren kommen im Prinzip alle dem Fachmann bekannten und/oder nach bekannten Methoden herstellbaren Polyarylether in Betracht. Entsprechende Methoden zur Herstellung werden weiter unten erläutert.

Bevorzugte Polyarylether sind aus Bausteinen der allgemeinen Formel (I) aufgebaut: 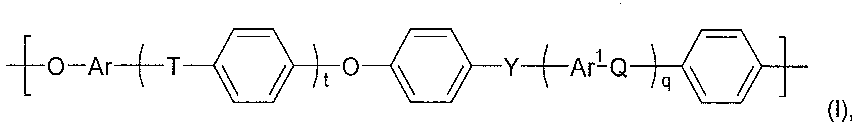 wobei die Symbole t, q, Q, T, Y, Ar und Ar¹ folgende Bedeutungen aufweisen:
- t, q:: unabhängig voneinander 0, 1, 2 oder 3,
- Q, T, Y:: unabhängig voneinander jeweils eine chemische Bindung oder Gruppe, ausgewählt aus -O-, -S-, -SO₂-, S=O, C=O, -N=N- und -CR^{a}R^{b}-, wobei R^{a} und R^{b} unabhängig voneinander jeweils für ein Wasserstoffatom oder eine C₁-C₁₂-Alkyl-, C₁-C₁₂-Alkoxy- oder C₆-C₁₈-Arylgruppe stehen, und wobei wenigstens eines aus Q, T und Y für -SO₂- steht und
- Ar, Ar¹:: unabhängig voneinander eine Arylengruppe mit von 6 bis 18 Kohlenstoffatomen.

Falls Q, T oder Y unter den oben genannten Voraussetzungen eine chemische Bindung ist, dann ist darunter zu verstehen, dass die links benachbarte und die rechts benachbarte Gruppe direkt miteinander über eine chemische Bindung verknüpft vorliegen.

Vorzugsweise werden Q, T und Y in Formel (I) allerdings unabhängig voneinander ausgewählt aus -O- und -SO₂-, mit der Maßgabe, dass wenigstens eines aus der Gruppe bestehend aus Q, T und Y für -SO₂- steht. Diese Polyarylether sind Polyarylethersulfone.

Gegenstand der vorliegenden Erfindung ist somit auch ein Verfahren, bei dem der Polyarylether ein Polyarylethersulfon ist.

Sofern Q, T oder Y -CR^{a}R^{b}- sind, stehen R^{a} und R^{b} unabhängig voneinander jeweils für ein Wasserstoffatom oder eine C₁-C₁₂-Alkyl-, C₁-C₁₂-Alkoxy- oder C₆-C₁₈-Arylgruppe.

Bevorzugte C₁-C₁₂-Alkylgruppen umfassen lineare und verzweigte, gesättigte Alkylgruppen mit von 1 bis 12 Kohlenstoffatomen. Insbesondere sind folgende Reste zu nennen: C₁-C₆-Alkylrest, wie Methyl, Ethyl, n-Propyl, i-Propyl, n-Butyl, sek-Butyl, 2- oder 3-Methyl-pentyl und längerkettige Reste wie unverzweigtes Heptyl, Octyl, Nonyl, Decyl, Undecyl, Lauryl und die ein- oder mehrfach verzweigten Analoga davon.

Als Alkylreste in den vorgenannten einsetzbaren C₁-C₁₂-Alkoxygruppen kommen die weiter oben definierten Alkylgruppen mit von 1 bis 12 Kohlenstoffatomen in Betracht. Vorzugsweise verwendbare Cycloalkylreste umfassen insbesondere C₃-C₁₂-Cycloalkylreste, wie zum Beispiel Cyclopropyl, Cyclobutyl, Cyclopentyl, Cyclohexyl, Cycloheptyl, Cyclooctyl, Cyclopropylmethyl, Cyclopropylethyl, Cyclopropylpropyl, Cyclobutylmethyl, Cyclobutylethyl, Cyclpentylethyl, -propyl, -butyl, -pentyl, -hexyl, Cyclohexylmethyl, -dimethyl, und -trimethyl.

Ar und Ar¹ bedeuten unabhängig voneinander eine C₆-C₁₈-Arylengruppe. Ausgehend von den weiter unten beschriebenen Ausgangsprodukten ist Ar vorzugsweise abgeleitet von einer elektronenreichen, leicht elektrophil angreifbaren aromatischen Substanz, die bevorzugt aus der Gruppe bestehend aus Hydrochinon, Resorcin, Dihydroxynaphthalin, insbesondere 2,7-Dihydroxynaphthalin, und 4,4'-Bisphenol ausgewählt wird. Vorzugsweise ist Ar¹ eine unsubstituierte C₆- oder C₁₂-Arylengruppe.

Als C₆-C₁₈-Arylengruppen Ar und Ar¹ kommen insbesondere Phenylengruppen, wie 1,2-, 1,3- und 1,4-Phenylen, Naphthylengruppen, wie beispielsweise 1,6-, 1,7-, 2,6- und 2,7-Naphthylen, sowie die von Anthracen, Phenanthren und Naphthacen abgeleiteten Arylengruppen in Betracht.

Vorzugsweise werden Ar und Ar¹ in der bevorzugten Ausführungsform gemäß Formel (I) unabhängig voneinander ausgewählt aus der Gruppe bestehend aus 1,4-Phenylen, 1,3-Phenylen, Naphthylen, insbesondere 2,7-Dihydroxynaphthylen, und 4,4'-Bisphenylen.

Bevorzugte Polyarylether sind solche, die mindestens eine der folgenden Bausteine Ia bis Io als wiederkehrende Struktureinheiten enthalten:

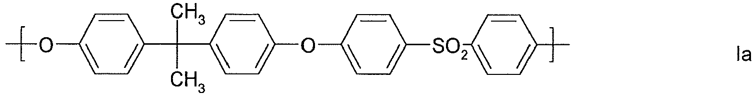

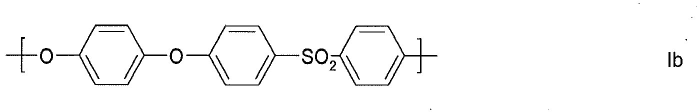

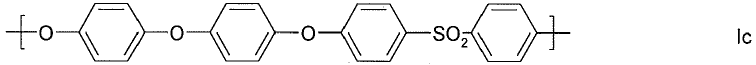

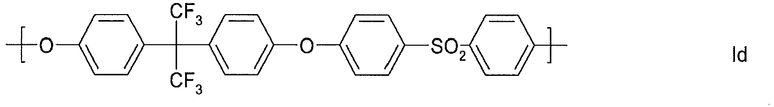

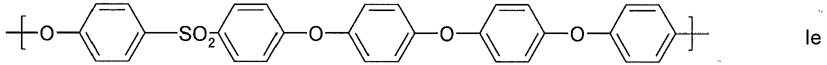

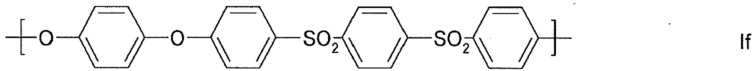

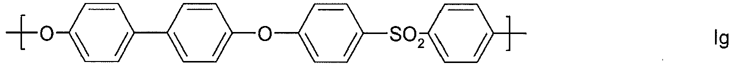

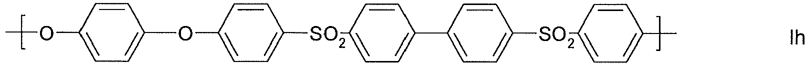

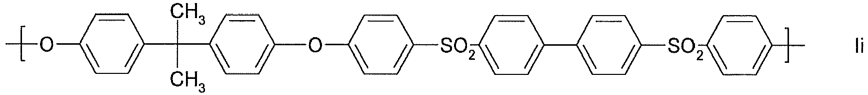

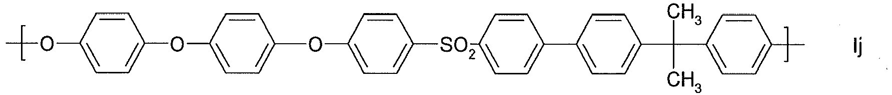

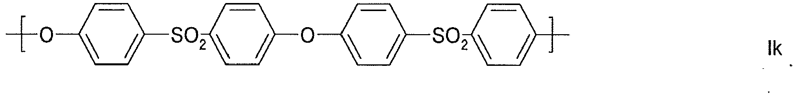

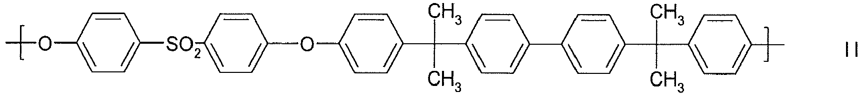

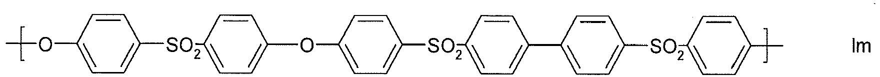

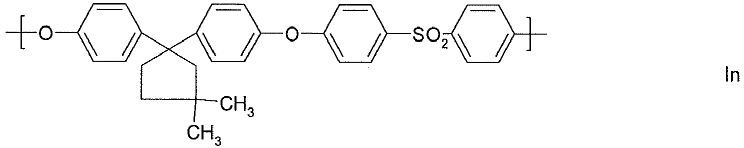

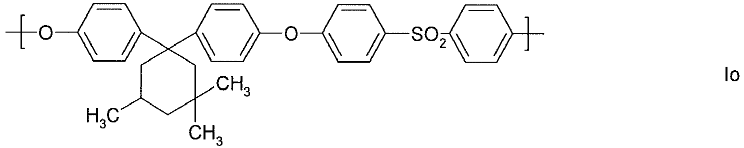

Zusätzlich zu den bevorzugten Bausteinen Ia bis Io sind auch solche Bausteine bevorzugt, in denen eine oder mehrere 1,4-Phenyleneinheiten, die von Hydrochinon abstammen, durch 1,3-Phenyleneinheiten, die von Resorcin abstammen, oder durch Naphthyleneinheiten, die von Dihydroxynaphthalin abstammen, ersetzt sind.

Als Bausteine der allgemeinen Formel (I) besonders bevorzugt sind die Bausteine Ia, Ig und Ik. Es ist außerdem besonders bevorzugt, wenn die Polyarylether im Wesentlichen aus einer Sorte Bausteine der allgemeinen Formel (I), insbesondere aus einem Baustein ausgewählt aus Ia, Ig und Ik aufgebaut sind.

In einer besonders bevorzugten Ausführungsform ist Ar = 1,4-Phenylen, t = 1, q = 0, T ist eine chemische Bindung und Y = SO₂. Aus der vorgenannten Wiederholungseinheit aufgebaute besonders bevorzugte Polyarylethersulfone werden als Polyphenylensulfon (PPSU) bezeichnet (Formel Ig).

In einer weiteren besonders bevorzugten Ausführungsform ist Ar = 1,4-Phenylen, t = 1, q = 0, T = C(CH₃)₂ und Y = SO₂. Aus der vorgenannten Wiederholungseinheit aufgebaute besonders bevorzugte Polyarylethersulfone werden als Polysulfon (PSU) bezeichnet (Formel Ia).

In einer weiteren besonders bevorzugten Ausführungsform ist Ar = 1,4-Phenylen, t = 1, q = 0, T = Y = SO₂. Aus der vorgenannten Wiederholungseinheit aufgebaute besonders bevorzugte Polyarylethersulfone werden als Polyethersulfon (PESU) bezeichnet (Formel Ik).

Abkürzungen wie PPSU, PSU und PESU entsprechen im Rahmen der vorliegenden Erfindung der DIN EN ISO 1043-1 (Kunststoffe - Kennbuchstaben und Kurzzeichen - Teil 1: Basis-Polymere und ihre besonderen Eigenschaften (ISO 1043-1:2001); Deutsche Fassung EN ISO 1043-1:2002).

Die Polyarylether weisen vorzugsweise gewichtsmittlere Molekulargewichte M_{w} von 10.000 bis 150.000 g/mol, insbesondere von 15.000 bis 120.000 g/mol, besonders bevorzugt von 18.000 bis 100.000 g/mol auf, bestimmt mittels Gelpermeationschromatographie im Lösungsmittel Dimethylacetamid gegen engverteiltes Polymethylmethacrylat als Standard.

Die Polyarylether weisen vorzugsweise ein zahlenmittleres Molekulargewicht Mₙ von 10 000 bis 35 000 g/mol auf, bestimmt mittels Gelpermeationschromatographie im Lösungsmittel Dimethylacetamid gegen engverteiltes Polymethylmethacrylat als Standard.

Die Polydispersität beträgt bevorzugt von 1,9 bis 7,5, besonders bevorzugt von 2,1 bis 4.

Darüber hinaus weisen die Polyarylether in Reinsubstanz vorzugsweise eine scheinbare Schmelzviskosität bei 350 °C/1150 s⁻¹ von 100 bis 1 000 Pa s, bevorzugt von 150 bis 300 Pa s und insbesondere bevorzugt von 150 bis 275 Pa s auf.

Die Schmelzeviskosität wurde mittels eines Kapillarrheometers bestimmt. Dabei wurde die scheinbare Viskosität bei 350°C als Funktion der Scherrate in einem Kapillarviskosimeter (Göttfert Kapillarviskosimeter Rheograph 2003) mit einer Kreiskapillare der Länge 30 mm, einem Radius von 0,5 mm, einem Einlaufwinkel der Düse von 180°, einem Durchmesser des Reservoirgefäßes der Schmelze von 12 mm und mit einer Vorheizzeit von 5 Minuten bestimmt. Angegeben sind die bei 1150 s⁻¹ bestimmten Werte.

Die Erweichungstemperatur T_{E} des Polyarylethers liegt üblicherweise im Bereich von 150 bis 230 °C, bevorzugt im Bereich von 155 bis 230 °C und insbesondere bevorzugt im Bereich von 160 bis 180 °C, bestimmt mittels dynamischer Differenzkalorimetrie. Verfahren hierzu sind dem Fachmann bekannt.

Unter der Erweichungstemperatur T_{E} des Polyarylethers wird vorliegend die Glasübergangstemperatur des reinen Polyarylethers, der 2 bis 30 Gew.-% des Extraktionsmittels (E), bezogen auf das Gesamtgewicht des Polyarylethers und des Extraktionsmittels (E), enthält, verstanden, wobei der Polyarylether kein Salz (S) enthält.

Bevorzugt wird unter der Erweichungstemperatur T_{E} des Polyarylethers vorliegend die Glasübergangstemperatur des reinen Polyarylethers, der 15 Gew.-% des Extraktionsmittels (E) bezogen auf das Gesamtgewicht des Polyarylethers und des Extraktionsmittels (E) enthält, verstanden, wobei der Polyarylether kein Salz (S) enthält.

Die Erweichungstemperatur T_{E} des Polyarylethers kann daher analog zur Glasübergangstemperatur T_{G} des Polyarylethers bestimmt werden.

Es versteht sich von selbst, dass die Erweichungstemperatur T_{E} des Polyarylethers unterhalb der Glasübergangstemperatur T_{G} des Polyarylethers liegt.

Gegenstand der vorliegenden Erfindung ist somit auch ein Verfahren, bei dem die Erweichungstemperatur T_{E} des Polyarylethers im Bereich von 150 bis 230 °C liegt.

Die Glasübergangstemperatur T_{G} des Polyarylethers liegt üblicherweise im Bereich von 160 bis 270 °C, bevorzugt im Bereich von 170 bis 250 °C und insbesondere bevorzugt im Bereich von 180 bis 230 °C, bestimmt durch Differenzthermoanalyse (DTA; differential calorimetry; DSC).

Verfahren zur Bestimmung der Glasübergangstemperatur T_{G} durch Differenzthermoanalyse sind dem Fachmann als solche bekannt.

Unter der Glasübergangstemperatur T_{G} wird die Temperatur verstanden, bei der der Polyarylether beim Abkühlen zu einem glasartigen Feststoff erstarrt.

Gegenstand der vorliegenden Erfindung ist somit auch ein Verfahren, bei dem die Glasübergangstemperatur T_{G} des Polyarylethers im Bereich von 160 °C bis 270 °C liegt.

Die Schmelztemperatur T_{M} des Polyarylethers liegt üblicherweise im Bereich von 200 bis 300 °C und bevorzugt im Bereich von 230 bis 280 °C, bestimmt durch Differenzthermoanalyse (DTA; Differential Scanning Calorimetry; DSC).

Unter der Schmelztemperatur T_{M} des Polyarylethers wird die Temperatur verstanden, bei der ein teilkristalliner Polyarylether vollständig vom festen Aggregatzustand in den flüssigen Aggregatzustand übergeht und der Polyarylether somit vollständig als Schmelze vorliegt.

Für den Fachmann ist es klar, dass bei einem amorphen Polyether die Schmelztemperatur T_{M} des Polyarylethers gleich der Glasübergangstemperatur T_{G} des Polyarylethers ist,

Herstellungsverfahren, die zu den vorgenannten Polyarylethern führen, sind dem Fachmann an sich bekannt und beispielsweise in Herman F. Mark, "Encyclopedia of Polymer Science and Technology", third edition, Volume 4, 2003, Kapitel "Polysulfones" auf den Seiten 2 bis 8 sowie in Hans R. Kricheldorf, "Aromatic Polyethers" in: Handbook of Polymer Synthesis, second edition, 2005 auf den Seiten 427 bis 443 beschrieben.

Polyarylether werden bevorzugt hergestellt durch die Umsetzung einer Komponente (a1) enthaltend mindestens eine aromatische Dihydroxyverbindung und einer Komponente (a2) enthaltend mindestens eine aromatische Sulfonverbindung mit zwei Halogensubstituenten. Das molare Verhältnis der Komponenten (a1) zu (a2) liegt bevorzugt im Bereich von 0,99 bis 1,4, besonders bevorzugt im Bereich von 1,0 bis 1,2 und am meisten bevorzugt im Bereich von 1,0 bis 1,1.

Die Umsetzung wird üblicherweise in Gegenwart einer Carbonatverbindung (C) durchgeführt.

Komponente (a1) enthält mindestens eine aromatische Dihydroxyverbindung. Komponente (a1) umfasst insbesondere folgende Verbindungen:
- 4,4' Dihydroxybiphenyl;
- Dihydroxybenzole, insbesondere Hydrochinon und Resorcin;
- Dihydroxynaphthaline, insbesondere 1,5-Dihydroxynaphthalin, 1,6-Dihydroxynaphthalin, 1,7- Dihydroxynaphthalin, und 2,7- Dihydroxynaphthalin;
- Andere Dihydroxybiphenyle als 4,4'-Biphenol, insbesondere 2,2'-Biphenol;
- Bisphenylether, insbesondere Bis(4-hydroxyphenyl)ether und Bis(2-hydroxyphenyl)ether;
- Bisphenylpropane, insbesondere 2,2-Bis(4-hydroxyphenyl)propan, 2,2-Bis(3-methyl-4-hydroxyphenyl)propan, und 2,2-Bis(3,5-dimethyl-4-hydroxyphenyl) propan;
- Bisphenylmethane, insbesondere Bis(4-hydroxyphenyl)methan
- Bisphenylcyclohexane, insbesondere Bis(4-hydroxyphenyl)-2,2,4-trimethylcyclohexan;
- Bisphenylsulfone, insbesondere Bis(4-hydroxyphenyl)sulfon;
- Bisphenylsulfide, insbesondere Bis(4-hydroxyphenyl)sulfid;
- Bisphenylketone, insbesondere Bis(4-hydroxyphenyl)keton;
- Bisphenylhexafluoropropane, insbesondere 2,2-Bis(3,5-dimethyl-4-hydroxyphenyl)hexafluoropropan; und
- Bisphenylfluorene, insbesondere 9,9-Bis(4-hydroxyphenyl)fluoren.

Vorzugsweise enthält die Komponente (a1) mindestens 50 Gew.-%, mehr bevorzugt mindestens 60 Gew.-%, besonders bevorzugt mindestens 80 Gew.-% und insbesondere mindestens 95 Gew.-% mindestens einer Dihydroxykomponente ausgewählt aus der Gruppe bestehend aus 4,4'-Dihydroxybiphenyl, 2,2-Bis(4-hydroxyphenyl)propan und Bis(4-hydroxyphenyl)sulfon, jeweils bezogen auf das Gesamtgewicht der Komponente (a1). Ganz besonders bevorzugt besteht die Komponente (a1) aus mindestens einer Dihydroxykomponente ausgewählt aus der Gruppe bestehend aus 4,4'-Dihydroxybiphenyl, 2,2-Bis(4-hydroxyphenyl)propan und Bis(4-hydroxyphenyl)sulfon.

2,2-Bis(4-hydroxyphenyl)propan ist auch bekannt unter dem Namen Bisphenol A. Bis(4-hydroxyphenyl)sulfon ist auch bekannt unter dem Namen Bisphenol S.

Vorzugsweise enthält die Komponente (a2) mindestens 50 Gew.-%, bevorzugt mindestens 60 Gew.-%, besonders bevorzugt mindestens 80 Gew.-% und insbesondere mindestens 95 Gew.-% mindestens einer aromatischen Sulfonverbindung mit zwei Halogensubstituenten, jeweils bezogen auf das Gesamtgewicht der Komponente (a2).

Als Komponente (a2) geeignete aromatische Sulfonverbindungen mit zwei Halogensubstituenten sind dem Fachmann grundsätzlich bekannt. Bevorzugte Komponenten (a2) sind insbesondere Dihalogendiphenylsulfone wie 4,4'-Dichlordiphenylsulfon, 4,4'-Difluordiphenylsulfon, 4,4'-Dibromdiphenylsulfon, 2,2'-Dichlordiphenylsulfon und 2,2'-Difluordiphenylsulfon. 4,4'-Dichlordiphenylsulfon und 4,4'-Difluordiphenylsulfon sind besonders bevorzugt. Ganz besonders bevorzugt ist 4,4'-Dichlordiphenylsulfon.

Die Umsetzung von 4,4'-Dihydroxybiphenyl als Komponente (a1) und 4,4'-Dihalogendiphenylsulfon als Komponente (a2) liefert Polyphenylensulfon (PPSU) als Polyarylethersulfon (Formel Ig).

Die Umsetzung von Bisphenol A als Komponente (a1) und 4,4'-Dihalogendiphenylsulfon als Komponente (a2) liefert Polysulfon (PSU) als Polyarylethersulfon (Formel Ia).

Die Umsetzung von Bisphenol S als Komponente (a1) und 4,4'-Dihalogendiphenylsulfon als Komponente (a2) liefert Polyethersulfon (PESU) als Polyarylethersulfon (Formel Ik).

### Bevorzugt als Polyarylethersulfon ist Polyphenylensulfon (PPSU) und Polyethersulfon (PESU)

Die Polyarylether können mehrere verschiedene Endgruppen aufweisen. Beispielsweise können sie Hydroxidendgruppen, Halogenendgruppen und/oder Alkoholatendgruppen aufweisen. Werden die Polyarylether im Anschluss an den Herstellungsprozess mit einem Veretherungsmittel umgesetzt, können die Polyarylether auch Etherendgruppen aufweisen. Geeignete Veretherungsmittel sind dem Fachmann bekannt und beispielsweise organische Monohalogenverbindungen.

Bevorzugte Veretherungsmittel sind ausgewählt aus der Gruppe bestehend aus Chlormethan, Brommethan, lodmethan und Dimethylcarbonat.

Geeignete Carbonatverbindungen (C) sind dem Fachmann als solche bekannt. Bevorzugte Carbonatverbindungen (C) sind Alkalimetallcarbonate und/oder Erdalkalimetallcarbonate. Vorzugsweise sind die Carbonatverbindungen (C) wasserfrei. Geeignete Carbonatverbindungen (C) sind insbesondere wasserfreies Alkalimetallcarbonat, vorzugsweise wasserfreies Natriumcarbonat, wasserfreies Kaliumcarbonat oder Mischungen davon, wobei wasserfreies Kaliumcarbonat ganz besonders bevorzugt ist.

Die Herstellung des salzhaltigen Polymers (sP), das den Polyarylether und das Salz (S) enthält, kann in Anwesenheit eines Lösungs- oder Verdünnungsmittels erfolgen, ebenso ist die Herstellung in Abwesenheit eines Lösungs- oder Verdünnungsmittels möglich. Bevorzugt ist die Herstellung in Abwesenheit eines Lösungs- oder Verdünnungsmittels. Besonders bevorzugt ist die Herstellung in Abwesenheit eines Lösungs- oder Verdünnungsmittels als Schmelzepolymerisationsverfahren.

Verfahren zur Herstellung von Polyarylethern in Anwesenheit eines Lösungs- oder Verdünnungsmittels sind dem Fachmann als solche bekannt. Sie können in einer erfindungsgemäßen Ausführungsform auch zur Herstellung des salzhaltigen Polymers (sP) verwendet werden. Dazu werden die Komponente (a1) und die Komponente (a2) in einem aprotisch-polaren Lösungsmittel in Gegenwart einer Carbonatverbindung (C) umgesetzt. Gegebenenfalls kann das Lösungsmittel außerdem ein Schleppmittel enthalten, das mit dem bei der Kondensationsreaktion entstehenden Wasser ein Azeotrop bildet. Geeignete aprotisch-polare Lösungsmittel sind beispielsweise ausgewählt aus der Gruppe bestehend aus N,N-Dimethylformamid, N,N-Dimethylacetamid, N-Methylpyrrolidon, N-Ethylpyrrolidon, Dimethylsulfoxid, Dimethylsulfon, Sulfolan und Diphenylsulfon. Geeignete Schleppmittel sind beispielsweise Toluol und/oder Chlorbenzol.

Die so hergestellten salzhaltigen Polymere (sP) können dann beispielsweise in Wasser nach dem Fachmann bekannten Methoden gefällt werden.

In einer bevorzugten Ausführungsform werden die salzhaltigen Polymere (sP) in Abwesenheit von Lösungs- oder Verdünnungsmitteln hergestellt. Sie werden besonders bevorzugt in einem Schmelzepolymerisationsverfahren hergestellt. Schmelzepolymerisationsverfahren für Polyarylether sind beispielsweise in der DE 2 749 645 und in der WO 2014/033321 beschrieben und können in einer bevorzugten Ausführungsform der vorliegenden Erfindung zur Herstellung des salzhaltigen Polymers (sP) eingesetzt werden.

Gegenstand der vorliegenden Erfindung ist somit auch ein Verfahren, bei dem das salzhaltige Polymer (sP) durch ein Schmelzepolymerisationsverfahren hergestellt wird.

Die Schmelzepolymerisation kann als Batchverfahren oder als kontinuierliches Verfahren durchgeführt werden. Bevorzugt ist die Durchführung als kontinuierliches Verfahren.

Geeignete Reaktoren sind sämtliche bekannten Reaktorformen, die zur Mischung von hochviskosen Materialien geeignet sind und außerdem eine Entfernung gasförmiger Kondensationsprodukte sowie ein Aufheizen der Monomere über deren Schmelzpunkt ermöglichen. Als Reaktoren bevorzugt sind Extruder oder Mischkneter, besonders bevorzugt sind Mischkneter. Bevorzugt sind außerdem Ein- oder Doppelwellenkneter, wobei Doppelwellenkneter besonders bevorzugt sind. Es ist weiterhin bevorzugt, dass der Mischkneter zusätzlich mit einem Rückflusskondensator ausgestattet ist, um flüchtiges Monomer, das bei den Reaktionstemperaturen gegebenenfalls verdampft, in den Mischkneter zurückzuführen.

Üblicherweise wird die Schmelzepolymerisation bei einer Temperatur durchgeführt, die unterhalb der Zersetzungstemperatur des Polyarylethers liegt. Vorzugsweise liegt die Temperatur bei der Schmelzepolymerisation mindestens 1 °C, bevorzugt mindestens 5°C und insbesondere bevorzugt mindestens 10 °C unterhalb der Zersetzungstemperatur des Polarylethers.

Im Allgemeinen wird die Schmelzepolymerisation bei einer Temperatur im Bereich von 200 bis 400 °C, bevorzugt im Bereich von 250 bis 350 °C durchgeführt.

In einer Ausführungsform werden die Komponente (a1) und die Komponente (a2) in einem molaren Verhältnis von 0,9 bis 1,4, bevorzugt von 1,0 bis 1,2 und insbesondere bevorzugt von 1,0 bis 1,1 in dem Mischkneter vorgelegt. Die Carbonatverbindung (C) wird dann als separate Komponente zugegeben. Bevorzugt wird die Carbonatverbindung (C) in einem molaren Verhältnis zur Komponente (a1) von 0,9 bis 1,22, bevorzugt von 1,0 bis 1,12 und insbesondere bevorzugt von 1,03 bis 1,10 zugeführt.

Werden die Komponente (a1) und die Komponente (a2) in dem Mischkneter vorgelegt, so ist es bevorzugt, dass die Komponenten (a1) und (a2) zunächst aufgeschmolzen werden und dann die Carbonatverbindung (C) zugeführt wird. Vorzugsweise werden die Komponenten (a1) und (a2) miteinander gemischt und aufgeschmolzen und dann erst dem Mischkneter zugeführt.

Es ist außerdem möglich, die Carbonatverbindung (C) mit einer der beiden Komponenten (a1) oder (a2) vorzulegen und anschließend die zweite der beiden Komponenten (a1) oder (a2) zuzugeben. Es ist insbesondere bevorzugt, die Carbonatverbindungen (C) mit der Komponente (a1) vorzulegen. Im Allgemeinen wird dann zunächst die Komponente (a1) mit der Carbonatverbindung (C) unter Bildung eines Dialkoholats umgesetzt und anschließend die Komponente (a2) zugegeben.

Bezüglich des molaren Verhältnisses der beiden Komponenten (a1) und (a2) sowie der Carbonatverbindung (C) gelten die vorher beschriebenen Ausführungen und Bevorzugungen, auch wenn die Carbonatverbindung (C) mit einer der beiden Komponenten (a1) oder (a2) vorgelegt wird.

Die Komponente (a1) und/oder (a2) kann flüssig oder fest in den Mischkneter eingebracht werden.

In einer weiteren Ausführungsform der vorliegenden Erfindung werden die Komponenten (a1) und (a2) und die Carbonatverbindung (C) zunächst als Pulver gemischt und dann dem Mischkneter zugeführt. In dem Mischkneter werden sie dann aufgeschmolzen und umgesetzt.

Die Umsetzungszeit im Reaktor beträgt im Allgemeinen 0,5 bis 3,5 Stunden, bevorzugt 1 bis 2 Stunden.

In einer weiteren Ausführungsform beträgt die Umsetzungszeit im Reaktor 0,5 bis 3,5 Stunden, bevorzugt 1,5 bis 3 Stunden.

Bei der Umsetzung der Komponente (a1) mit der Komponente (a2) in Gegenwart der Carbonatverbindung (C) werden als Kondensationsprodukte neben dem Polyarylether Wasser, Kohlenstoffdioxid und das Salz (S) gebildet. Das gebildete Wasser und das gebildete Kohlenstoffdioxid können während der Umsetzung als gasförmige Bestandteile aus dem Reaktor entfernt werden. Das Salz (S) verbleibt im Allgemeinen im Polyarylether unter Erhalt des salzhaltigen Polymers (sP). Im Allgemeinen handelt es sich bei dem Salz (S) um ein anorganisches Salz, wenn als Carbonatverbindung (C) eine anorganische Carbonatverbindung (C) eingesetzt wird. Bevorzugt handelt es sich bei dem Salz (S) um ein Alkalimetallhalogenid, wenn als Carbonatverbindung (C) ein Alkalimetallcarbonat eingesetzt wird. Am meisten bevorzugt handelt es sich bei dem Salz (S) um Kaliumchlorid und/oder Natriumchlorid, wenn als Carbonatverbindung (C) Kaliumcarbonat und/oder Natriumcarbonat eingesetzt wird.

Gegenstand der vorliegenden Erfindung ist somit auch ein Verfahren, bei dem das Salz (S) ein anorganisches Salz enthält.

Gegenstand der vorliegenden Erfindung ist weiterhin ein Verfahren, bei dem das Salz (S) Kaliumchlorid und/oder Natriumchlorid enthält.

Das Salz (S) besitzt im Allgemeinen eine Partikelgröße im Bereich von 0,1 bis 100 µm, bevorzugt im Bereich von 0,5 bis 50 µm, besonders bevorzugt im Bereich von 0,8 bis 30 µm und am meisten bevorzugt im Bereich von 1 bis 10 µm. Die Partikelgröße wird durch SEM (Scanning Electron Microscopy; Rasterelektronenmikroskopie)-Aufnahmen bei einer Beschleunigungsspannung von 8 kV bestmmt.

Das Salz (S) liegt im Allgemeinen partikulär dispers verteilt in dem salzhaltigen Polymer (sP) vor.

### Schritt a)

In Schritt a) wird das salzhaltige Polymer (sP) bei einer ersten Temperatur T₁, die oberhalb der Erweichungstemperatur T_{E} des Polyarylethers liegt, bereitgestellt.

Bevorzugt liegt die erste Temperatur T₁ oberhalb der Glasübergangstemperatur T_{G} des Polyarylethers.

Die erste Temperatur T₁ liegt üblicherweise mindestens 1 °C, bevorzugt mindestens 5°C und insbesondere bevorzugt mindestens 10 °C oberhalb der Erweichungstemperatur T_{E} des Polyarylethers.

In einer weiteren bevorzugten Ausführungsform liegt die erste Temperatur T₁ mindestens 1 °C, bevorzugt mindestens 5 °C und insbesondere bevorzugt mindestens 10 °C oberhalb der Glasübergangstemperatur T_{G} des Polyarylethers.

Die erste Temperatur T₁ liegt unterhalb der Zersetzungstemperatur des Polyarylethers. Bevorzugt liegt die erste Temperatur T₁ mindestens 1 °C, bevorzugt mindestens 5°C und insbesondere bevorzugt mindestens 10 °C unterhalb der Zersetzungstemperatur des Polyarylethers.

In einer bevorzugten Ausführungsform liegt die erste Temperatur T₁ in einem Bereich von 160 bis 300 °C, bevorzugt im Bereich von 200 bis 280 °C und insbesondere bevorzugt im Bereich von 220 bis 260 °C.

Gegenstand der vorliegenden Erfindung ist somit auch ein Verfahren, bei dem die erste Temperatur T₁ in Schritt a) in einem Bereich von 160 bis 300 °C liegt.

Erfindungsgemäß außerdem bevorzugt liegt die erste Temperatur T₁ im Bereich von 1 bis 100 °C, bevorzugt im Bereich von 5 bis 50 °C und insbesondere bevorzugt im Bereich von 20 bis 50 °C oberhalb der Glasübergangstemperaur T_{G} des Polyarylethers.

Gegenstand der vorliegenden Erfindung ist somit auch ein Verfahren, bei dem die erste Temperatur T₁ im Bereich von 1 bis 100 °C oberhalb der Erweichungstemperatur T_{E} des Polyarylethers liegt.

Die erste Temperatur T₁ kann außerdem oberhalb der Schmelztemperatur T_{M} des Polyarylethers liegen. Wenn die erste Temperatur T₁ oberhalb der Schmelztemperatur T_{M} des Polyarylethers liegt, wird das salzhaltige Polymer (sP) in Schritt a) als Schmelze bereitgestellt.

In Schritt a) kann das salzhaltige Polymer (sP) in jedem beliebigen Reaktor bereitgestellt werden, der es ermöglicht, das salzhaltige Polymer (sP) bei der ersten Temperatur T₁ zu halten. Derartige Reaktoren sind dem Fachmann bekannt. Geeignete Reaktoren sind beispielsweise Rührkesselreaktoren, Autoklaven, Kneter, Extruder oder Dünnschichtverdampfer.

Der Reaktor kann außerdem dynamische oder statische Mischorgane umfassen. Dynamische und statische Mischorgane als solche sind dem Fachmann bekannt. Dynamische Mischorgane sind beispielsweise Rührer wie Propellerrührer, Schaufelrührer, Ankerrührer und selbstreinigende Doppelwellenmischorgane.

Das salzhaltige Polymer (sP) kann in Schritt a) nach allen dem Fachmann bekannten Methoden bereitgestellt werden. Beispielsweise kann das salzhaltige Polymer (sP) zunächst in einem kontinuierlichen oder einem Batchverfahren wie vorstehend beschrieben hergestellt werden und anschließend zu festem Pulver, beispielsweise in Form von Granulat oder Pulver, verarbeitet werden. Das salzhaltige Polymer (sP) kann dann in Schritt a) bereitgestellt werden, indem das feste Pulver des salzhaltigen Polymers (sP) auf die erste Temperatur T₁ erwärmt wird.

Darüber hinaus ist es möglich, das salzhaltige Polymer (sP) direkt im Anschluss an seine Herstellung, bevorzugt an die Herstellung durch ein Schmelzepolymerisationsverfahren, insbesondere bevorzugt an die Herstellung durch Schmelzepolymerisation, in einem kontinuierlichen Verfahren in Schritt a) bereitzustellen. Im Allgemeinen wird das salzhaltige Polymer (sP) dann direkt im Anschluss an seine Herstellung in Schritt a) bereitgestellt, bevorzugt indem die bei der Herstellung erhaltene Schmelze des salzhaltigen Polymers (sP) in Schritt a) bereitgestellt wird, ohne das salzhaltige Polymer (sP) zuvor zu einem festen Pulver zu verarbeiten. Diese Ausführungsform ist bevorzugt.

### Schritt b)

In Schritt b) wird das in Schritt a) bereitgestellte salzhaltige Polymer (sP) mit einem Extraktionsmittel (E) in Kontakt gebracht und ein entsalztes Polymer (eP), das den Polyarylether enthält, und ein salzhaltiges Extraktionsmittel (sE), das das Extraktionsmittel (E) und das Salz (S) enthält, erhalten.

Bei Schritt b) handelt es sich um eine Extraktion. Im Folgenden werden die Begriffe "Schritt b)" und "Extraktion" daher synonym verwendet.

Bevorzugt wird Schritt b) direkt im Anschluss an Schritt a) durchgeführt.

Als Extraktionsmittel (E) kann genau ein Extraktionsmittel eingesetzt werden. Ebenso ist es möglich, eine Mischung aus zwei oder mehreren Extraktionsmitteln einzusetzen.

Als Extraktionsmittel (E) eignet sich prinzipiell jedes Lösungsmittel, das das Salz (S) löst. Bevorzugt enthält das Extraktionsmittel (E) ein protisches Lösungsmittel. Besonders bevorzugt enthält das Extraktionsmittel (E) Wasser.

Gegenstand der vorliegenden Erfindung ist somit auch ein Verfahren, bei dem als Extraktionsmittel (E) ein protisches Lösungsmittel verwendet wird.

Im Allgemeinen enthält das Extraktionsmittel (E) mindestens 50 Gew.-%, bevorzugt mindestens 70 Gew.-%, insbesondere bevorzugt mindestens 80 Gew.-% und am meisten bevorzugt mindestens 90 Gew.-% Wasser, jeweils bezogen auf das Gesamtgewicht des Extraktionsmittels (E).

Gegenstand der vorliegenden Erfindung ist somit auch ein Verfahren, bei dem das Extraktionsmittel (E) in Schritt b) Wasser enthält.

Das salzhaltige Polymer (sP) wird im Allgemeinen in einem Reaktor mit dem Extraktionsmittel (E) in Kontakt gebracht. Dazu eignen sich prinzipiell alle dem Fachmann bekannten Reaktorformen, die zum Einsatz bei den in Schritt b) verwendeten Drücken und Temperaturen geeignet sind. Beispielsweise wird das salzhaltige Polymer (sP) in Schritt b) in dem gleichen Reaktor mit dem Extraktionsmittel (E) in Kontakt gebracht, in dem das salzhaltige Polymer (sP) bei der ersten Temperatur T₁ in Schritt a) bereitgestellt wurde.

Für den Reaktor in Schritt b) gelten dann die zuvor für den Reaktor in Schritt a) beschriebenen Ausführungen und Bevorzugungen. Bevorzugt wird das salzhaltige Polymer (sP) in Schritt b) mit dem Extraktionsmittel (E) kontinuierlich in einem dynamischen Mischer in Kontakt gebracht. Verfahren hierzu sind dem Fachmann bekannt.

Das salzhaltige Polymer (sP) wird in Schritt b) vorzugsweise bei einer zweiten Temperatur T₂, die oberhalb der Erweichungstemperatur T_{E}, vorzusweise oberhalb der Glasübergangstemperatur T_{G} des Polyarylethers liegt, mit dem Extraktionsmittel (E) in Kontakt gebracht.

Üblicherweise liegt die zweite Temperatur T₂ mindestens 1 °C, bevorzugt mindestens 5°C und insbesondere bevorzugt mindestens 10 °C oberhalb der Erweichungstemperatur T_{E}, vorzugsweise der Glasübergangstemperaur T_{G} des Polyarylethers.

Gegenstand der vorliegenden Erfindung ist somit auch ein Verfahren, bei dem das salzhaltige Polymer (sP) in Schritt b) bei einer zweiten Temperatur T₂, die oberhalb der Erweichungstemperatur T_{E} des Polyarylethers liegt, mit dem Extraktionsmittel (E) in Kontakt gebracht wird.

Die zweite Temperatur T₂ liegt unterhalb der Zersetzungstemperatur des Polyarylethers. Bevorzugt liegt die zweite Temperatur T₂ mindestens 1 °C, bevorzugt mindestens 5°C und insbesondere bevorzugt mindestens 10 °C unter der Zersetzungstemperatur des Polyarylethers.

Die zweite Temperatur T₂, bei der das salzhaltige Polymer (sP) in Schritt b) mit dem Extraktionsmittel (E) in Kontakt gebracht wird, liegt beispielsweise im Bereich von 160 bis 300 °C, bevorzugt im Bereich von 200 bis 280 °C und insbesondere bevorzugt im Bereich von 220 bis 260 °C.

Gegenstand der vorliegenden Erfindung ist somit auch ein Verfahren, bei dem die zweite Temperatur T₂, bei der das salzhaltige Polymer (sP) in Schritt b) mit dem Extraktionsmittel (E) in Kontakt gebracht wird, im Bereich von 160 bis 300 °C liegt.

Die zweite Temperatur T₂ kann oberhalb der ersten Temperatur T₁ liegen, ebenso kann die zweite Temperatur T₂ unterhalb der ersten Temperatur T₁ liegen, jeweils unter der Bedingung, dass die erste Temperatur T₁ und die zweite Temperatur T₂ oberhalb der Erweichungstemperatur T_{E}, vorzugsweise oberhalb der Glasübergangstemperatur T_{G} und unterhalb der Zersetzungstemperatur des Polyarylethers liegen. In einer Ausführungsform des erfindungsgemäßen Verfahrens ist die zweite Temperatur T₂ gleich der ersten Temperatur T₁.

Gegenstand der vorliegenden Erfindung ist somit auch ein Verfahren, bei dem in Schritt b) das in Schritt a) bereitgestellte salzhaltige Polymer (sP) bei der ersten Temperatur T₁ mit dem Extraktionsmittel (E) in Kontakt gebracht wird. Diese Ausführungsform ist bevorzugt.

Der Druck in Schritt b) liegt üblicherweise im Bereich von 6 bis 100 bar, bevorzugt im Bereich von 10 bis 70 bar und insbesondere bevorzugt im Bereich von 20 bis 50 bar.

Gegenstand der vorliegenden Erfindung ist somit auch ein Verfahren, bei dem in Schritt b) das in Schritt a) bereitgestellte salzhaltige Polymer (sP) bei einem Druck im Bereich von 6 bis 100 bar mit dem Extraktionsmittel (E) in Kontakt gebracht wird.

In Schritt b) werden das entsalzte Polymer (eP) und das salzhaltige Extraktionsmittel (sE) erhalten.

Das in Schritt b) erhaltene salzhaltige Extraktionsmittel (sE) enthält den Teil des Salzes (S), der aus dem salzhaltigen Polymer (sP) entfernt worden ist. Im Allgemeinen enthält das salzhaltige Extraktionsmittel (sE) 0,1 bis 20 Gew.-% des Salzes (S), bevorzugt 0,5 bis 10Gew.-% und insbesondere bevorzugt 1 bis 5 Gew.-% des Salzes (S), jeweils bezogen auf das Gesamtgewicht des salzhaltigen Extraktionsmittels (sE).

In einer weiteren bevorzugten Ausführungsform der vorliegenden Erfindung umfasst die Bereitstellung des salzhaltigen Polymers (sP) in Verfahrensschritt a) die folgenden Schritte i) bis iv):
i) Bereitstellen eines ersten salzhaltigen Polymers (s1P), das den Polyarylether und das Salz (S) enthält,
ii) Granulieren des in Schritt i) bereitgestellten ersten salzhaltigen Polymers (s1P) unter Erhalt eines granulierten ersten salzhaltigen Polymers (gs1P),
iii) Inkontaktbringen des in Schritt ii) erhaltenen granulierten ersten salzhaltigen Polymers (gs1P) mit dem Extraktionsmittel (E) unter Erhalt des salzhaltigen Polymers (sP), das den Polyarylether und Reste des Salzes (S) enthält, und eines ersten salzhaltigen Extraktionsmittels (sE1), das das Extraktionsmittel (E) und einen Teil des Salzes (S) enthält,
iv) Erwärmen des in Schritt iii) erhaltenen salzhaltigen Polymers (sP) auf eine erste Temperatur T₁, die oberhalb der Erweichungstemperatur T_{E}, vorzugsweise oberhalb der Glasübergangstemperatur T des Polyarylethers liegt,
und Verfahrensschritt b) den folgenden Schritt v):
v) Inkontaktbringen des in Schritt iv) erwärmten salzhaltigen Polymers (sP) mit dem Extraktionsmittel (E) unter Erhalt des entsalzten Polymers (eP), das den Polyarylether enthält, und eines zweiten salzhaltigen Extraktionsmittels (sE2), das das Extraktionsmittel (E) und die Reste des Salzes (S) enthält.

Gegenstand der vorliegenden Erfindung ist somit außerdem ein Verfahren, das die folgenden Schritte umfasst:
i) Bereitstellen eines ersten salzhaltigen Polymers (s1 P), das den Polyarylether und das Salz (S) enthält,
ii) Granulieren des in Schritt i) bereitgestellten ersten salzhaltigen Polymers (s1P) unter Erhalt eines granulierten ersten salzhaltigen Polymers (gs1P),
iii) Inkontaktbringen des in Schritt ii) erhaltenen granulierten ersten salzhaltigen Polymers (gs1P) mit dem Extraktionsmittel (E) unter Erhalt des salzhaltigen Polymers (sP), das den Polyarylether und Reste des Salzes (S) enthält, und eines ersten salzhaltigen Extraktionsmittels (sE1), das das Extraktionsmittel (E) und einen Teil des Salzes (S) enthält,
iv) Erwärmen des in Schritt iii) erhaltenen salzhaltigen Polymers (sP) auf eine erste Temperatur T₁, die oberhalb der Erweichungstemperatur T_{E} des Polyarylethers liegt,
v) Inkontaktbringen des in Schritt iv) erwärmten salzhaltigen Polymers (sP) mit dem Extraktionsmittel (E) unter Erhalt des entsalzten Polymers (eP), das den Polyarylether enthält, und eines zweiten salzhaltigen Extraktionsmittels (sE2), das das Extraktionsmittel (E) und die Reste des Salzes (S) enthält.

Enthält das erste salzhaltige Polymer (s1P) weniger als 10 Gew.-% des Salzes (S), bevorzugt weniger als 8 Gew.-% und insbesondere bevorzugt weniger als 6 Gew.-%, bezogen auf das Gesamtgewicht des ersten salzhaltigen Polymers (s1P), so müssen die Schritte i) bis iv) nicht durchgeführt werden. Stattdessen werden im Allgemeinen nur die Schritte a) und b) durchgeführt. In dieser Ausführungsform ist das erste salzhaltige Polymer (s1P) gleich dem salzhaltigen Polymer (sP).

Enthält das erste salzhaltige Polymer (s1 P) dagegen mindestens 6 Gew.-%, bevorzugt mindestens 8 Gew.-% und insbesondere bevorzugt mindestens 10 Gew.-% des Salzes (S), so werden im Allgemeinen die Schritte i) bis iv) durchgeführt. Das erste salzhaltige Polymer (s1P) ist dann von dem salzhaltigen Polymer (sP) verschieden.

In Schritt i) wird das erste salzhaltige Polymer (s1P) bereitgestellt. Verfahren zur Bereitstellung des ersten salzhaltigen Polymers (s1P) sind dem Fachmann als solche bekannt. Vorzugsweise wird das erste salzhaltige Polymer (s1P) in Schritt i) durch ein Schmelzepolymerisationsverfahren bereitgestellt.

Gegenstand der vorliegenden Erfindung ist somit auch ein Verfahren, bei dem das erste salzhaltige Polymer (s1P) in Schritt i) durch ein Schmelzepolymerisationsverfahren bereitgestellt wird.

Für das Schmelzepolymerisationsverfahren gelten die zuvor beschriebenen Ausführungen und Bevorzugungen.

Für das erste salzhaltige Polymer (s1P) gelten die vorstehenden Ausführungen und Bevorzugungen zum salzhaltigen Polymer (sP) entsprechend. Wenn das erste salzhaltige Polymer (s1P) von dem salzhaltigen Polymer (sP) verschieden ist, wenn also die Verfahrensschritte i) bis iii) durchgeführt werden, so enthält das erste salzhaltige Polymer (s1P) mehr Salz (S) als das salzhaltige Polymer (sP).

In Schritt ii) wird das in Schritt i) bereitgestellte erste salzhaltige Polymer (s1P) unter Erhalt des granulierten ersten salzhaltigen Polymers (gs1P) granuliert. Verfahren hierzu sind dem Fachmann als solche bekannt.

Vorzugsweise erfolgen Schritt i) und Schritt ii) kontinuierlich. In dieser Ausführungsform wird das erste salzhaltige Polymer (s1P) bevorzugt durch ein Schmelzepolymerisationsverfahren bereitgestellt, wobei der zur Schmelzepolymerisation eingesetzte Reaktor einen Extruder umfasst, durch den das erste salzhaltige Polymer (s1P) im Anschluss an Schritt i) extrudiert und dann gemäß Schritt ii) granuliert werden kann. Die Granulierung kann beispielsweise als Stranggranulierung oder als Unterwassergranulierung erfolgen.

In Schritt ii) wird das erste salzhaltige Polymer (s1P) beispielsweise auf eine Partikelgröße im Bereich von 0,3 bis 10 mm, bevorzugt im Bereich von 0,4 bis 6 mm und insbesondere bevorzugt im Bereich von 0,5 bis 2 mm granuliert.

Das granulierte erste salzhaltige Polymer (gs1P) weist somit im Allgemeinen eine Partikelgröße im Bereich von 0,3 bis 10 mm, bevorzugt im Bereich von 0,4 bis 6 mm und insbesondere bevorzugt im Bereich von 0,5 bis 2 mm auf, bestimmt durch Bildanalyse.

Gegenstand der vorliegenden Erfindung ist somit auch ein Verfahren, bei dem das erste salzhaltige Polymer (s1P) in Schritt ii) auf eine Partikelgröße im Bereich von 0,3 bis 10 mm granuliert wird.

In Schritt iii) wird das in Schritt ii) erhaltene granulierte erste salzhaltige Polymer (gs1P) mit dem Extraktionsmittel (E) in Kontakt gebracht. Für das Extraktionsmittel (E) gelten die gleichen Ausführungen und Bevorzugungen wie für Schritt b) beschrieben.

Schritt iii) wird auch als "Vorextraktion" bezeichnet. Die Begriffe "Schritt iii)" und "Vorextraktion" werden im Folgenden synonym gebraucht.

Schritt iii) wird üblicherweise bei einer Temperatur unterhalb der Glasübergangstemperatur T_{G} des Polyarylethers durchgeführt. Bevorzugt wird Schritt iii) unterhalb der Erweichungstemperatur T_{E} des Polyarylethers durchgeführt.

Schritt iii) wird im Allgemeinen bei einer Temperatur im Bereich von 50 bis 150 °C, bevorzugt im Bereich von 60 bis 100 °C und insbesondere bevorzugt im Bereich von 70 bis 100 °C durchgeführt.

Der Absolutdruck im Reaktor während des Verfahrensschritts iii) liegt bevorzugt im Bereich von 1 bis 10 bar, besonders bevorzugt im Bereich von 1 bis 7 bar, am meisten bevorzugt im Bereich von 1 bis 5 bar.

Geeignete Reaktoren für Schritt iii) sind dem Fachmann als solche bekannt. Geeignete Reaktoren sind beispielsweise Rührkesselreaktoren und Rohrreaktoren. Erfindungsgemäß bevorzugt sind Rohrreaktoren.

Es ist außerdem bevorzugt, dass der in Schritt iii) eingesetzte Reaktor von außen geheizt werden kann auf die Temperatur, bei der das granulierte erste salzhaltige Polymer (gs1P) mit dem Extraktionsmittel (E) in Kontakt gebracht wird.

Erfindungsgemäß kann der Reaktor gegebenenfalls auch beispielsweise mit Zentrifugen und/oder Filtern ausgestattet sein, um das in Schritt iii) erhaltene erste salzhaltige Extraktionsmittel (sE1) von dem in Schritt iii) erhaltenen salzhaltigen Polymer (sP) abzutrennen.

Das granulierte erste salzhaltige Polymer (gs1P) kann in dem Reaktor als Festbett vorliegen, so dass es sich bei dem eingesetzten Reaktor um einen Festbettreaktor handelt. Ebenso ist es möglich und erfindungsgemäß bevorzugt, in Schritt iii) einen Gegenstromreaktor einzusetzen.

Gegenstromreaktoren sind dem Fachmann als solche bekannt. In einer Ausführungsform der vorliegenden Erfindung kann das granulierte erste salzhaltige Polymer (gs1P) beispielsweise kontinuierlich durch den Gegenstromreaktor geströmt werden und aus entgegengesetzter Richtung das Extraktionsmittel (E) zugeführt werden.

Wird Schritt iii) in einem Festbettreaktor durchgeführt, so wird das Extraktionsmittel (E) hindurchgeleitet. Im Allgemeinen wird das Extraktionsmittel (E) von unten nach oben durch den Reaktor geleitet oder von oben nach unten. Bevorzugt wird das Extraktionsmittel (E) von unten nach oben durch den Reaktor geleitet.

Wird ein Gegenstromreaktor verwendet, so wird im Allgemeinen das granulierte erste salzhaltige Polymer (gs1P) kontinuierlich von oben in den Reaktor gegeben und unten aus diesem entfernt, während gleichzeitig von unten das Extraktionsmittel (E) in den Reaktor geführt wird und oben herausströmt.

In Schritt iii) wird das salzhaltige Polymer (sP), das den Polyarylether und Reste des Salzes (S) enthält, und ein erstes salzhaltiges Extraktionsmittel (sE1), das das Extraktionsmittel (E) und einen Teil des Salzes (S) enthält, erhalten.

Das erste salzhaltige Extraktionsmittel (sE1) enthält das Extraktionsmittel (E) und den Teil des Salzes (S), der aus dem granulierten ersten salzhaltigen Polymer (gs1P) entfernt wurde. Im Allgemeinen enthält das erste salzhaltige Extraktionsmittel (sE1) 0,09 bis 18 Gew.-% des Salzes (S), bevorzugt 0,45 bis 9 Gew.-% des Salzes (S) und insbesondere bevorzugt 0,9 bis 5 Gew.-% des Salzes (S), jeweils bezogen auf das Gesamtgewicht des ersten salzhaltigen Extraktionsmittels (sE1).

Unter "Reste des Salzes (S)" werden erfindungsgemäß 0,02 bis 10 Gew.-%, bevorzugt 0,1 bis 8 Gew.-% und insbesondere bevorzugt 0,2 bis 6 Gew.-% des Salzes (S) verstanden, jeweils bezogen auf das Gesamtgewicht des salzhaltigen Polymers (sP).

Anders ausgedrückt enthält das in Schritt iii) erhaltene salzhaltige Polymer (sP) im Allgemeinen 0,02 bis 10 Gew.-%, bevorzugt 0,1 bis 8 Gew.-% und insbesondere bevorzugt 0,2 bis 6 Gew.-% des Salzes (S), jeweils bezogen auf das Gesamtgewicht des salzhaltigen Polymers (sP).

Es versteht sich von selbst, dass das in Schritt iii) erhaltene salzhaltige Polymer (sP) weniger Salz (S) als das erste salzhaltige Polymer (s1P) und das granulierte erste salzhaltige Polymer (gs1P) enthält.
Enthält das erste salzhaltige Polymer (s1P) weniger als 10 Gew.-%, bevorzugt weniger als 8 Gew.-% und insbesondere bevorzugt weniger als 6 Gew.-% des Salzes (S), bezogen auf das Gesamtgewicht des ersten salzhaltigen Polymers (s1P), werden die Schritte i) bis iii) im Allgemeinen nicht durchgeführt.

Das erste salzhaltige Polymer (s1P) wird dann direkt als salzhaltiges Polymer (sP) eingesetzt.

In Schritt iv) wird das in Schritt iii) erhaltene salzhaltige Polymer (sP) auf eine erste Temperatur T₁, die oberhalb der Erweichungstemperatur T_{E}, bevorzugt oberhalb der Glasübergangstemperatur T_{G} des Polyarylethers liegt, erwärmt.

In Schritt iv) wird das salzhaltige Polymer (sP) im Allgemeinen in einem Reaktor erwärmt. Als Reaktor in Schritt iv) eignen sich die gleichen Reaktoren, wie sie oben für Schritt a) beschrieben worden sind. Es gelten daher für die Reaktoren in Schritt iv) die gleichen Ausführungen und Bevorzugungen wie für die Reaktoren in Schritt a).

Das Erwärmen des salzhaltigen Polymers (sP) kann nach allen dem Fachmann bekannten Methoden erfolgen.

Für die erste Temperatur T₁, auf die das salzhaltige Polymer (sP) in Schritt iv) erwärmt wird, gelten die zuvor für die erste Temperatur T₁ in Schritt a) beschriebenen Ausführungen und Bevorzugungen.

In Schritt v) wird das in Schritt iv) erwärmte salzhaltige Polymer (sP) mit dem Extraktionsmittel (E) in Kontakt gebracht. Für das Inkontaktbringen des in Schritt iv) erwärmten salzhaltigen Polymers (sP) mit dem Extraktionsmittel (E) gelten die gleichen Ausführungen und Bevorzugungen wie vorstehend für Schritt b) beschrieben.

In Schritt v) wird das entsalzte Polymer (eP), das den Polyarylether enthält, und ein zweites salzhaltiges Extraktionsmittel (sE2), das das Extraktionsmittel (E) und die Reste des Salzes (S) enthält, erhalten.

Es versteht sich von selbst, dass das zweite salzhaltige Extraktionsmittel (sE2), das in Schritt v) erhalten wird, gleich dem salzhaltigen Extraktionsmittel (sE), das in Schritt b) erhalten wird, ist, wenn das erste salzhaltige Polymer (s1P) gleich dem salzhaltigen Polymer (sP) ist.

Das zweite salzhaltige Extraktionsmittel (sE2) kann in einer Ausführungsform der vorliegenden Erfindung als Extraktionsmittel (E) in Verfahrensschritt iii) eingesetzt werden.

Es versteht sich von selbst, dass das entsalzte Polymer (eP), das in Schritt b) bzw. in Schritt v) erhalten wird, weniger Salz (S) enthält als das erste salzhaltige Polymer (s1P) und das salzhaltige Polymer (sP). Im Allgemeinen enthält das entsalzte Polymer (eP) noch Spuren des Salzes (S).

Unter "Spuren des Salzes (S)" wird vorliegend ein Salzgehalt im entsalzten Polymer (eP) von maximal 150 Gew.-ppm, bevorzugt maximal 100 Gew.-ppm, insbesondere bevorzugt maximal 80 Gew.-ppm und am meisten bevorzugt maximal 50 Gew.-ppm des Salzes (S) verstanden, jeweils bezogen auf das Gesamtgewicht des entsalzten Polymers (eP).

Im Allgemeinen enthält das entsalzte Polymer (eP) 0,01 bis 150 Gew.-ppm des Salzes (S), bevorzugt 0,1 bis 100 Gew.-ppm, besonders bevorzugt 1 bis 80 Gew.-ppm und insbesondere 5 bis 50 Gew.-ppm des Salzes (S), jeweils bezogen auf das Gesamtgewicht des entsalzten Polymers (eP).

In einer Ausführungsform der vorliegenden Erfindung enthält das entsalzte Polymer (eP) maximal 150 Gew.-ppm, bevorzugt maximal 100 Gew.-ppm, insbesondere bevorzugt maximal 80 Gew.-ppm und am meisten bevorzugt maximal 50 Gew.-ppm des Salzes (S), jeweils bezogen auf das Gesamtgewicht des entsalzten Polymers (eP).

Gegenstand der vorliegenden Erfindung ist somit auch ein Verfahren, bei dem das in Schritt b) erhaltene entsalzte Polymer (eP) maximal 150 Gew.-ppm des Salzes (S) enthält, bezogen auf das Gesamtgewicht des entsalzten Polymers (eP).

Die Untergrenze des Gehalts an Salz (S) im entsalzten Polymer (eP) liegt im Allgemeinen bei 0,01 Gew.-ppm, bevorzugt bei 0,1 Gew.-ppm, besonders bevorzugt bei 1 Gew.-ppm und insbesondere bevorzugt bei 5 Gew.-ppm.

In einer insbesondere bevorzugten Ausführungsform ist das entsalzte Polymer (eP) im Wesentlichen frei von dem Salz (S). "Im Wesentlichen frei" bedeutet im Rahmen der vorliegenden Erfindung, dass das entsalzte Polymer (eP) maximal 15 Gew.-ppm, bevorzugt maximal 10 Gew.-ppm und insbesondere bevorzugt maximal 5 Gew.-ppm des Salzes (S) enthält.

Schritt b) kann in einer Ausführungsform der vorliegenden Erfindung wiederholt werden. Er kann dabei einmal oder auch mehrmals wiederholt werden. Ebenso können Schritt iii) und Schritt v) einmal oder mehrmals wiederholt werden.

Das entsalzte Polymer (eP) kann von dem salzhaltigen Extraktionsmittel (sE) nach dem Fachmann bekannten Methoden abgetrennt werden. Beispielsweise kann es durch Sedimentieren von dem salzhaltigen Extraktionsmittel (sE) abgetrennt werden.

Es ist außerdem möglich, das entsalzte Polymer (eP) zu trocknen. Zur Trocknung eignen sich prinzipiell alle dem Fachmann bekannten Methoden. Beispielsweise kann das entsalzte Polymer (eP) bei erhöhten Temperaturen getrocknet werden. Bevorzugt sind Temperaturen im Bereich von 50 bis 160 °C, besonders bevorzugt im Bereich von 100 bis 150 °C. Die Temperatur beim Trocknen liegt üblicherweise unterhalb der Erweichungstemperatur T_{E} des Polyarylethers. Die Trocknung kann gegebenenfalls unter vermindertem Druck durchgeführt werden.

Für die Abtrennung des entsalzten Polymers (eP) von dem zweiten salzhaltigen Extraktionsmittel (sE2) gelten die vorstehend beschriebenen Ausführungen und Bevorzugungen zur Abtrennung des entsalzten Polymers (eP) von dem salzhaltigen Extraktionsmittel (sE).

Wenn das salzhaltige Polymer (sP) in einem Schmelzepolymerisationsverfahren hergestellt worden ist, enthält das salzhaltige Polymer (sP) und damit auch das entsalzte Polymer (eP) kein aprotisch-polares Lösungsmittel, das ausgewählt ist aus der Gruppe bestehend aus N,N-Dimethylformamid, N,N-Dimethylacetamid, N-Methylpyrrolidon, N-Ethylpyrrolidon, Dimethylsulfoxid, Dimethylsulfon, Sulfolan, Diphenylsulfon und Mischungen daraus, bevorzugt enthält das entsalzte Polymer (eP) kein aprotisch-polares Lösungsmittel.

Gegenstand der vorliegenden Erfindung ist somit auch ein entsalztes Polymer (eP), das 0 bis 100 Gew.-ppm, bevorzugt 0 bis 20 Gew.-ppm und insbesondere bevorzugt 0 bis 10 Gew.-ppm eines aprotisch-polaren Lösungsmittels enthält, ausgewählt aus der Gruppe bestehend aus N,N-Dimethylformamid, N,N-Dimethylacetamid, N-Methylpyrrolidon, N-Ethylpyrrolidon, Dimethylsulfoxid, Dimethylsulfon, Sulfolan, Diphenylsulfon und Mischungen daraus, am meisten bevorzugt kein aprotisch-polares Lösungsmittel enthält, und das weniger als 150 Gew.-ppm, bevorzugt weniger als 100 Gew.-ppm, mehr bevorzugt weniger als 80 Gew.-ppm und am meisten bevorzugt weniger als 50 Gew.-ppm des Salzes (S) enthält, wobei die Gewichts-ppm jeweils bezogen sind auf das Gesamtgewicht des entsalzten Polymers (eP).

Gegenstand der vorliegenden Erfindung ist somit auch ein entsalztes Polymer (eP) erhältlich nach dem erfindungsgemäßen Verfahren.

Gegenstand der vorliegenden Erfindung ist außerdem ein entsalztes Polymer (eP), erhältlich nach dem erfindungsgemäßen Verfahren, das 0 bis 100 Gew.-ppm eines aprotisch-polaren Lösungsmittels, ausgewählt aus der Gruppe bestehend aus N,N-Dimethylformamid, N,N-Dimethylacetamid, N-Methylpyrrolidon, N-Ethylpyrrolidon, Dimethylsulfoxid, Dimethylsulfon, Sulfolan, Diphenylsulfon und Mischungen daraus, enthält, und das weniger als 150 Gew.-ppm des Salzes (S) enthält, wobei die Gew.-ppm jeweils bezogen sind auf das Gesamtgewicht des entsalzten Polymers (eP).

Die nach dem erfindungsgemäßen Verfahren erhältlichen entsalzten Polymere (eP) weisen vorzugsweise eine scheinbare Schmelzviskosität bei 350 °C/1150 s⁻¹ von 100 bis 1000 Pa s, bevorzugt von 150 bis 300 Pa s und insbesondere bevorzugt von 150 bis 275 Pa s auf.

Die Schmelzeviskosität wurde mittels eines Kapillarrheometers bestimmt. Dabei wurde die scheinbare Viskosität bei 350°C als Funktion der Scherrate in einem Kapillarviskosimeter (Göttfert Kapillarviskosimeter Rheograph 2003) mit einer Kreiskapillare der Länge 30 mm, einem Radius von 0,5 mm, einem Einlaufwinkel der Düse von 180°, einem Durchmesser des Reservoirgefäßes der Schmelze von 12 mm und mit einer Vorheizzeit von 5 Minuten bestimmt. Angegeben sind die bei 1150 s⁻¹ bestimmten Werte.

Die Viskositätszahlen der nach dem erfindungsgemäßen Verfahren entsalzten Polymere (eP) liegen im Allgemeinen im Bereich von 20 bis 120 ml/g, bevorzugt von 30 bis 100 ml/g und insbesondere bevorzugt von 35 bis 95 ml/g, bestimmt durch Ubbelohde-Viskositätszahlmessung einer 0,01 g/ml Lösung des salzhaltigen Polymers (sP) in einer 1 : 1 Phenol/1,2-Dichlorbenzol-Mischung nach DIN 51562.

Gegenstand der vorliegenden Erfindung ist außerdem ein Verfahren zur Entsalzung eines salzhaltigen Polymers (sP), das einen Polyarylether mit einer Glasübergangstemperatur T_{G} und ein Salz (S) enthält, umfassend die Schritte
a) Bereitstellen des salzhaltigen Polymers (sP) bei einer ersten Temperatur T₁, die oberhalb der Glasübergangstemperatur T_{G} des Polyarylethers liegt,
b) in Kontakt bringen des in Schritt a) bereitgestellten salzhaltigen Polymers (sP) mit einem Extraktionsmittel (E) unter Erhalt eines entsalzten Polymers (eP), das den Polyarylether enthält, und eines salzhaltigen Extraktionsmittels (sE), das das Extraktionsmittel (E) und das Salz (S) enthält.

Nachfolgend wird die Erfindung anhand von Beispielen näher erläutert, ohne sie hierauf zu beschränken.

### Beispiele:

### Beispiel 1 und 2

Polyethersulfon (PESU, Ultrason E) wurde ausgehend von Bisphenol S als Komponente (a1) und 4,4'-Dihalogendiphenylsulfon als Komponente (a2) in Gegenwart von Kaliumcarbonat als Carbonatkomponente (C) in einem Schmelzepolymerisationsverfahren hergestellt.

Das erhaltene Polyethersulfon wurde granuliert und bei 80 °C für 24 Stunden mit Wasser extrahiert. Der Salzgehalt des so erhaltenen salzhaltigen Polymes (sP) lag bei weniger als 1% der ursprünglichen Konzentration des Salzes (S). Anschließend wurde das Salz (S) (Kaliumchlorid) aus dem erhaltenen salzhaltigen Polymer (sP) bei einer Temperatur von 250°C mit Wasser im Autoklaven extrahiert. Es wurden jeweils 30 g salzhaltiges Polymer (sP) mit Wasser extrahiert.

Das Wasser wurde für die verschiedenen Beispiele nach unterschiedlichen Zeiträumen gewechselt. In Beispiel 1 wurde das Wasser nach 7, 23, 29 und 48 Stunden gewechselt, in Beispiel 2 wurde das Wasser alle zwei Stunden gewechselt.

Der resultierende Salzgehalt des entsalzten Polymers (eP) in Abhängigkeit von der Extraktionszeit aus Beispiel 1 ist in Tabelle 1 angegeben und aus Beispiel 2 in Tabelle 2. Ebenso ist in den Tabellen 1 und 2 die Leitfähigkeit des Wassers, mit dem das Salz (S) extrahiert wurde, angegeben

**Tabelle 1**

| Zeit [h] | Leitfähigkeit [mS] | Chlor [g/100g] | Chlorid {g/100g] | Kalium [g/100g] |
|---|---|---|---|---|
| 8 | 16,18 | | | |
| 25 | 1,72 | 0,24 | 0,042 | 0,09 |
| 31 | 0,24 | | | |
| 49 | 0,21 | 0,17 | 0,01 | 0,013 |

**Tabelle 2**

| Zeit [h] | Leitfähigkeit [mS] | Chlor [g/100g] | Chlorid {g/100g] | Kalium [g/100g] |
|---|---|---|---|---|
| 2 | 16,15 | | | |
| 4 | 4,12 | | | |
| 6 | 0,70 | | | |
| 8 | 0,24 | | | |
| 10 | 0,11 | | | |
| 12 | 0,04 | | | |
| 14 | 0,04 | 0,17 | 0,002 | 0,037 |

## Patentansprüche

1. Verfahren zur Entsalzung eines salzhaltigen Polymers (sP), das einen Polyarylether mit einer Erweichungstemperatur T_{E} und ein Salz (S) enthält, umfassend die Schritte
a) Bereitstellen des salzhaltigen Polymers (sP) bei einer ersten Temperatur T₁, die oberhalb der Erweichungstemperatur T_{E} des Polyarylethers liegt,
b) Inkontaktbringen des in Schritt a) bereitgestellten salzhaltigen Polymers (sP) mit einem Extraktionsmittel (E) unter Erhalt eines entsalzten Polymers (eP), das den Polyarylether enthält, und eines salzhaltigen Extraktionsmittels (sE), das das Extraktionsmittel (E) und das Salz (S) enthält,
wobei der Schritt a) die folgenden Schritte umfasst:
i) Bereitstellen eines ersten salzhaltigen Polymers (s1P), das den Polyarylether und das Salz (S) enthält,
ii) Granulieren des in Schritt i) bereitgestellten ersten salzhaltigen Polymers (s1P) unter Erhalt eines granulierten ersten salzhaltigen Polymers (gs1P),
iii) Inkontaktbringen des in Schritt ii) erhaltenen granulierten ersten salzhaltigen Polymers (gs1P) mit dem Extraktionsmittel (E) unter Erhalt des salzhaltigen Polymers (sP), das den Polyarylether und Reste des Salzes (S) enthält, und eines ersten salzhaltigen Extraktionsmittels (sE1), das das Extraktionsmittel (E) und einen Teil des Salzes (S) enthält,
iv) Erwärmen des in Schritt iii) erhaltenen salzhaltigen Polymers (sP) auf eine erste Temperatur T₁, die oberhalb der Erweichungstemperatur T_{E}, vorzugsweise oberhalb der Glasübergangstemperatur T_{G} des Polyarylethers liegt,
und wobei der Schritt b) den folgenden Schritt umfasst:
v) Inkontaktbringen des in Schritt iv) erwärmten salzhaltigen Polymers (sP) mit dem Extraktionsmittel (E) unter Erhalt des entsalzten Polymers (eP), das den Polyarylether enthält, und eines zweiten salzhaltigen Extraktionsmittels (sE2), das das Extraktionsmittel (E) und die Reste des Salzes (S) enthält,
wobei das erste salzhaltige Polymer (s1P) in Schritt i) durch ein Schmelzepolymerisationsverfahren bereitgestellt und die Erweichingstemperatur (T_{E}) und die Glasübergangstemperatur (T_{G}) werden mittels dynamischer Differenzkalorimetrie (DKK; differential scanning calorimetrie DSC) bestimmt.

2. Verfahren gemäß Anspruch 1, **dadurch gekennzeichnet, dass** das salzhaltige Polymer (sP) in Schritt b) bei einer zweiten Temperatur T₂, die oberhalb der Erweichungstemperatur T_{E} des Polyarylethers liegt, mit dem Extraktionsmittel (E) in Kontakt gebracht wird.

3. Verfahren gemäß Anspruch 2, **dadurch gekennzeichnet, dass** die zweite Temperatur T₂, bei der das salzhaltige Polymer (sP) in Schritt b) mit dem Extraktionsmittel (E) in Kontakt gebracht wird, im Bereich von 160 bis 300 °C liegt.

4. Verfahren gemäß einem der Ansprüche 1 bis 3, **dadurch gekennzeichnet, dass** die erste Temperatur T₁ in Schritt a) in einem Bereich von 160 bis 300 °C liegt.

5. Verfahren gemäß einem der Ansprüche 1 bis 4, **dadurch gekennzeichnet, dass** die erste Temperatur T₁ im Bereich von 1 bis 100 °C oberhalb der Erweichungstemperatur T_{E} des Polyarylethers liegt.

6. Verfahren gemäß einem der Ansprüche 1 bis 5, **dadurch gekennzeichnet, dass** die Erweichungstemperatur T_{E} des Polyarylethers im Bereich von 150 bis 230 °C liegt.

7. Verfahren gemäß einem der Ansprüche 1 bis 6, **dadurch gekennzeichnet, dass** das Extraktionsmittel (E) in Schritt b) Wasser enthält.

8. Verfahren gemäß einem der Ansprüche 1 bis 7, **dadurch gekennzeichnet, dass** das in Schritt b) erhaltene entsalzte Polymer (eP) maximal 150 Gew.-ppm des Salzes (S) enthält, bezogen auf das Gesamtgewicht des entsalzten Polymers (eP).

9. Verfahren gemäß einem der Ansprüche 1 oder 8, **dadurch gekennzeichnet, dass** das erste salzhaltige Polymer (s1P) in Schritt ii) auf eine Partikelgröße im Bereich von 0,3 bis 10 mm granuliert wird.

10. Verfahren gemäß einem der Ansprüche 1 bis 9, **dadurch gekennzeichnet, dass** der Polyarylether ein Polyarylethersulfon ist.

11. Verfahren gemäß einem der Ansprüche 1 bis 10, **dadurch gekennzeichnet, dass** das Salz (S) Kaliumchlorid und/oder Natriumchlorid enthält.

## Claims

1. A method for desalinating a salt-containing polymer (SP) comprising a polyaryl ether having a softening temperature T_{S} and a salt (S), comprising the steps of
a) providing the salt-containing polymer (SP) at a first temperature T₁ above the softening temperature T_{S} of the polyaryl ether,
b) contacting the salt-containing polymer (SP) provided in step a) with an extractant (E) to obtain a desalinated polymer (DP) comprising the polyaryl ether, and a salt-containing extractant (SE) comprising the extractant (E) and the salt (S),
wherein step a) comprises the following steps:
i) providing a first salt-containing polymer (SP1) comprising the polyaryl ether and the salt (S),
ii) pelletizing the first salt-containing polymer (SP1) provided in step i) to obtain a pelletized first salt-containing polymer (PSP1),
iii) contacting the pelletized first salt-containing polymer (PSP1) obtained in step ii) with the extractant (E) to obtain the salt-containing polymer (SP) comprising the polyaryl ether and residues of the salt (S), and a first salt-containing extractant (SE1) comprising the extractant (E) and a portion of the salt (S),
iv) heating the salt-containing polymer (SP) obtained in step iii) to a first temperature T₁ above the softening temperature T_{S}, preferably above the glass transition temperature T_{G}, of the polyaryl ether,
and wherein step b) comprises the following step:
v) contacting the salt-containing polymer (SP) heated in step iv) with the extractant (E) to obtain the desalinated polymer (DP) comprising the polyaryl ether, and a second salt-containing extractant (SE2) comprising the extractant (E) and the residues of the salt (S),
wherein the first salt-containing polymer (SP1) is provided in step i) by a melt polymerization method and the softening temperature (T_{S}) and the glass transition temperature (T_{G}) are determined by means of dynamic differential calorimetry (DDC; differential scanning calorimetry DSC).

2. The method according to claim 1, wherein the salt-containing polymer (SP) is contacted with the extractant (E) in step b) at a second temperature T₂ above the softening temperature T_{S} of the polyaryl ether.

3. The method according to claim 2, wherein the second temperature T₂ at which the salt-containing polymer (SP) is contacted with the extractant (E) in step b) is in the range from 160 to 300°C.

4. The method according to any of claims 1 to 3, wherein the first temperature T₁ in step a) is within a range from 160 to 300°C.

5. The method according to any of claims 1 to 4, wherein the first temperature T₁ is in the range from 1 to 100°C above the softening temperature T_{S} of the polyaryl ether.

6. The method according to any of claims 1 to 5, wherein the softening temperature T_{S} of the polyaryl ether is in the range from 150 to 230°C.

7. The method according to any of claims 1 to 6, wherein the extractant (E) in step b) comprises water.

8. The method according to any of claims 1 to 7, wherein the desalinated polymer (DP) obtained in step b) comprises not more than 150 ppm by weight of the salt (S), based on the total weight of the desalinated polymer (DP).

9. The method according to either of claims 1 and 8, wherein the first salt-containing polymer (SP1) is pelletized in step ii) to a particle size in the range from 0.3 to 10 mm.

10. The method according to any of claims 1 to 9, wherein the polyaryl ether is a polyaryl ether sulfone.

11. The method according to any of claims 1 to 10, wherein the salt (S) comprises potassium chloride and/or sodium chloride.

## Revendications

1. Procédé de dessalage d'un polymère contenant un sel (sP), qui contient un éther polyarylique ayant une température de ramollissement T_{E} et un sel (S), comprenant les étapes suivantes :
a) la préparation du polymère contenant un sel (sP) à une première température T₁, qui est supérieure à la température de ramollissement T_{E} de l'éther polyarylique,
b) la mise en contact du polymère contenant un sel (sP) préparé à l'étape a) avec un agent d'extraction (E) pour obtenir un polymère dessalé (eP), qui contient l'éther polyarylique, et un agent d'extraction contenant un sel (sE), qui contient l'agent d'extraction (E) et le sel (S),
l'étape a) comprenant les étapes suivantes :
i) la préparation d'un premier polymère contenant un sel (s1P), qui contient l'éther polyarylique et le sel (S),
ii) la granulation du premier polymère contenant un sel (s1P) préparé à l'étape i) pour obtenir un premier polymère contenant un sel granulé (gs1P),
iii) la mise en contact du premier polymère contenant un sel granulé (gs1P) obtenu à l'étape ii) avec l'agent d'extraction (E) pour obtenir le polymère contenant un sel (sP), qui contient l'éther polyarylique et des résidus du sel (S), et un premier agent d'extraction contenant un sel (sE1), qui contient l'agent d'extraction (E) et une partie du sel (S),
iv) le chauffage du polymère contenant un sel (sP) obtenu à l'étape iii) à une première température T₁, qui est supérieure à la température de ramollissement T_{E}, de préférence supérieure à la température de transition vitreuse T_{G} de l'éther polyarylique,
et l'étape b) comprenant l'étape suivante :
v) la mise en contact du polymère contenant un sel (sP) chauffé à l'étape iv) avec l'agent d'extraction (E) pour obtenir le polymère dessalé (eP), qui contient l'éther polyarylique, et un deuxième agent d'extraction contenant un sel (sE2), qui contient l'agent d'extraction (E) et les résidus du sel (S),
le premier polymère contenant un sel (s1P) préparé à l'étape i) par un procédé de polymérisation à l'état fondu, et la température de ramollissement (T_{E}) et la température de transition vitreuse (T_{G}) étant déterminées par calorimétrie différentielle dynamique (CDD ; differential scanning calorimetry DSC).

2. Procédé selon la revendication 1, **caractérisé en ce que** le polymère contenant un sel (sP) est mis en contact avec l'agent d'extraction (E) à l'étape b) à une deuxième température T₂ qui est supérieure à la température de ramollissement T_{E} de l'éther polyarylique.

3. Procédé selon la revendication 2, **caractérisé en ce que** la deuxième température T₂ à laquelle le polymère contenant un sel (sP) est mis en contact avec l'agent d'extraction (E) à l'étape b) se situe dans la plage allant de 160 à 300 °C.

4. Procédé selon l'une quelconque des revendications 1 à 3, **caractérisé en ce que** la première température T₁ à l'étape a) se situe dans une plage allant de 160 à 300 °C.

5. Procédé selon l'une quelconque des revendications 1 à 4, **caractérisé en ce que** la première température T₁ se situe dans la plage allant de 1 à 100 °C au-dessus de la température de ramollissement T_{E} de l'éther polyarylique.

6. Procédé selon l'une quelconque des revendications 1 à 5, **caractérisé en ce que** la température de ramollissement T_{E} de l'éther polyarylique se situe dans la plage allant de 150 à 230 °C.

7. Procédé selon l'une quelconque des revendications 1 à 6, **caractérisé en ce que** l'agent d'extraction (E) à l'étape b) contient de l'eau.

8. Procédé selon l'une quelconque des revendications 1 à 7, **caractérisé en ce que** le polymère dessalé (eP) obtenu à l'étape b) contient au plus 150 ppm en poids du sel (S), par rapport au poids total du polymère dessalé (eP).

9. Procédé selon l'une quelconque des revendications 1 ou 8, **caractérisé en ce que** le premier polymère contenant un sel (s1P) est granulé à l'étape ii) à une taille de particules dans la plage allant de 0,3 à 10 mm.

10. Procédé selon l'une quelconque des revendications 1 à 9, **caractérisé en ce que** l'éther polyarylique est une polyaryléther-sulfone.

11. Procédé selon l'une quelconque des revendications 1 à 10, **caractérisé en ce que** le sel (S) contient du chlorure de potassium et/ou du chlorure de sodium.
